# EUROPEAN PATENT SPECIFICATION

(11) **EP 4 076 232 B1**
(45) Date of publication and mention of the grant of the patent: **31.07.2024**
(21) Application number: 21724047.2
(22) Date of filing: 26.03.2021
(51) Int. Cl.: A61B 17/34, A61B 46/10, A61B 90/40, A61M 16/04, A61M 16/08, A61M 25/01, A61B 1/00, A61M 16/10

(54) **AN ADAPTER FOR A FACE MASK AND AN INSTRUMENT SHIELDING APPARATUS**
ADAPTER FÜR GESICHTSMASKE UND INSTRUMENTENABSCHIRMVORRICHTUNG
ADAPTATEUR POUR MASQUE FACIAL ET APPAREIL DE PROTECTION D'INSTRUMENT

(30) Priority: 26.03.2020 IE S20200050; 31.03.2020 IE S20200052; 02.07.2020 IE S20200150; 10.07.2020 IE S20200157
(43) Date of publication of application: 26.10.2022
(73) Proprietor: Palliare Limited, Dangan Galway (IE)
(72) Inventor: O'DEA, John, Galway (IE); BARRETT, Hilary Elizabeth, Limerick, V94R97R (IE); PERRY, Sophie Sarah Joan, Skibbereen County Cork (IE); MCCARTHY, Robert Gorby, Dublin A96 XE73 (IE)
(74) Representative: Gorman, Francis Fergus
(86) International application number: PCT/IE2021/000005
(87) International publication number: WO 2021/191884

(56) References cited:
- WO-A1-97/04828
- US-A- 4 691 702
- US-A- 5 101 817
- US-A- 5 545 169
- US-A- 5 582 165
- US-A1- 2003 188 750
- US-A1- 2004 221 842
- US-A1- 2007 113 854
- US-A1- 2013 296 653
- US-A1- 2014 128 678
- US-A1- 2018 207 389
- US-B1- 7 021 313

## Description

The present invention relates to an instrument shielding device for shielding an instrument being entered into a subject orally or nasally through a face mask, and also for shielding an instrument being entered through a trocar into a subject.

During an endoscopic procedure on a subject, which may be an investigative procedure or a surgical procedure, being carried out endoscopically, either orally or nasally, a surgeon and other healthcare personnel present during the carrying out of the procedure are subjected to any pathogens, including bacteria, infections and viruses exhaled by the subject. Additionally, if the procedure being carried out endoscopically requires insufflating of a vessel, lumen or cavity in the subject, in which the procedure is being carried out, a surgeon and other healthcare personnel present, during the carrying out of the procedure, are also subjected to any pathogens, bacteria, infections and viruses in the vessel, lumen or cavity of the subject, which may become entrained in insufflating gas leaking orally or nasally from the subject.

A further problem associated with the carrying out of an endoscopic procedure on a subject is that the endoscope, which must be gripped by hand by the surgeon carrying out the procedure, may become contaminated with pathogens, once inserted in the subject, and as the surgeon manoeuvres the endoscope inwardly and outwardly of the subject during the procedure, any such pathogen contamination of the endoscope can transfer to the hands or the gloved hands of the surgeon. This becomes a particularly serious problem during the withdrawal of the endoscope from the subject.

Additionally, similar problems arise in carrying out minimal invasive investigative and surgical procedures in a subject, such as, for example, procedures being carried out laparoscopically in a vessel, lumen or cavity of a subject which is being insufflated. In such cases, access is gained to the vessel, lumen or cavity through a trocar, or other such access device which is entered into the vessel, lumen or cavity through an incision formed, for example, in the abdominal wall of a subject. Instruments, including a laparoscope are entered into the vessel, lumen or cavity, for example, the peritoneal cavity through the trocar, and the peritoneal cavity is insufflated either through the trocar, or through another trocar or a Veress needle also entered into the peritoneal cavity through the abdominal wall. Insufflating gases insufflating the vessel, lumen or cavity, for example, the peritoneal cavity, tend to leak through the incision between the trocar and the vessel wall, particularly, as the trocar is being tilted or otherwise manoeuvred during the carrying out of the procedure. Insufflating gas leaking through the incision may carry pathogens, including bacteria, infections and viruses from the cavity to which the surgeon carrying out the procedure and other personnel involved in the carrying out of the procedure are subjected, with consequential risk to the surgeon and personnel.

Further, in carrying out such minimal invasive procedures, a laparoscope, and other instruments must be gripped by hand by the surgeon carrying out the procedure. The laparoscope and instruments may become contaminated with pathogens, bacteria, infections and viruses which may reside in the cavity in which the procedure is being carried out. As the surgeon manoeuvres such instruments inwardly and particularly, outwardly of the subject during the procedure, and in particular, on withdrawal of the instruments from the cavity, any pathogen contamination on the instruments is readily transferred to the hands or the gloved hands of the surgeon, this is also particularly undesirable, since it can also result in serious consequences for the surgeon.

There is therefore a need for apparatus to address at least some of these problems.

U.S. Specification No. 4,691,702 of Chantzis discloses an aspirating device which includes a flexible catheter and a shielding device for shielding the catheter. The shielding device comprises a housing defining a storage chamber within which a shielding sleeve is located. One end of the shielding sleeve is secured to the housing in the storage chamber, and the other end is secured to the catheter adjacent a distal end thereof, so that as the catheter is urged through the housing, the sleeve extends with the catheter from the housing to shield the catheter.

PCT Specification No. WO 97/04828 of Porat discloses a shielding device for shielding an instrument being entered nasally into a subject, the shielding device comprises a housing defining a storage chamber within which a shielding sleeve is located. One end of the shielding sleeve is secured to the housing in the storage chamber and the other end is secured to a proximal end of the instrument, so that as the instrument is being withdrawn from a subject, the instrument is drawn through the shielding sleeve.

The present invention is directed towards providing an instrument shielding device for shielding an instrument being entered into a subject orally or nasally through a face mask or for shielding an instrument being entered through a trocar into a subject which addresses at least some of the above problems.

According to the invention there is provided an instrument shielding device for shielding an instrument, the instrument shielding device comprising an elongated shielding sleeve extending between a first end and a second end and defining an elongated instrument accommodating lumen extending therethrough from the first end to the second end for accommodating the instrument therein, and a sleeve storing housing defining a storage chamber for storing the shielding sleeve in a stored state, one of the first and second ends of the shielding sleeve being sealably secured to the sleeve storing housing, a first coupling element adapted to couple the first end of the shielding sleeve to one of a face mask and a trocar, and a second coupling element adapted to couple the second end of the shielding sleeve to the instrument, wherein the sleeve storing housing comprises a tubular member extending through the storage chamber, and defining with the sleeve storing housing the storage chamber of annular shape extending around the tubular member for accommodating the shielding sleeve in the stored state with the tubular member extending into the instrument accommodating lumen of the shielding sleeve, and the tubular member defining an instrument accommodating bore extending through the sleeve storing housing for accommodating the instrument therethrough.

Preferably, the first coupling element is adapted to sealably couple the first end of the shielding sleeve to the one of the face mask and the trocar.

Advantageously, the first coupling element is adapted to releasably couple the first end of the shielding sleeve to the one of the face mask and the trocar.

In an alternative embodiment of the invention the first coupling element is adapted to non-releasably couple the first end of the shielding sleeve to the one of the face mask and the trocar.

In one embodiment of the invention the second coupling element is adapted to sealably couple the second end of the shielding sleeve to the instrument.

Advantageously, the second coupling element is adapted to releasably couple the second end of the shielding sleeve to the instrument.

Preferably, the second coupling element is adapted to couple the second end of the shielding sleeve to the instrument adjacent a proximal end of the instrument.

Preferably, the shielding sleeve is urgeable from a collapsed stored state, to an extended state for shielding the instrument.

Advantageously, the sleeve storing housing comprises a closure member for closing the storage chamber, the closure member having an access opening therethrough for accommodating the shielding sleeve therethrough from the storage chamber.

In one embodiment of the invention the access opening in the closure member is centrally aligned with the instrument accommodating bore extending through the sleeve storing chamber, and the tubular member extending through the storage chamber defines with a rim of the closure member defining the access opening therein, an annular access opening for accommodating the shielding sleeve from the storage chamber.

In another embodiment of the invention the closure member is releasably coupled to the sleeve storing housing.

In another embodiment of the invention the one of the first and second ends of the shielding sleeve, which is sealably secured to the sleeve storing housing is sealably secured to the sleeve storing housing in the storage chamber thereof.

In another embodiment of the invention the second end of the shielding sleeve is sealably secured to the sleeve storing housing, and the second coupling element is adapted for coupling the sleeve storing housing to the instrument.

In another embodiment of the invention the second coupling element is located in the sleeve storing housing.

In a further embodiment of the invention the second coupling element is located in the instrument accommodating bore extending through the sleeve storing housing.

In another embodiment of the invention the second coupling element is adapted to slideably engage the instrument.

In a further embodiment of the invention the second coupling element is adapted to tightly slideably engage the instrument.

Preferably, the second coupling element is adapted to releasably engage the instrument.

In one embodiment of the invention the second coupling element comprises one of a duckbill valve, a multi-leaf valve and a cross-slit valve.

In another embodiment of the invention the first coupling element is adapted to couple the first end of the shielding sleeve directly onto the one of the face mask and the trocar adjacent an instrument port thereof, or onto a port connector, the port connector being adapted for engaging the instrument port of the one of the face mask and the trocar.

Preferably, the first coupling element comprises a coupling ring adapted to engage the one of the face mask and the trocar adjacent the instrument port thereof or the port connector for securing the first end of the shielding sleeve thereto.

Advantageously, the coupling ring is adapted to engage an annular receiving groove extending around the instrument port of the one of the face mask and the trocar or the port connector with a portion of the shielding sleeve adjacent the first end thereof sealably engaged in and retained in the annular receiving groove by the coupling ring.

Advantageously, the coupling ring is located in the instrument accommodating lumen defined by the portion of the shielding sleeve adjacent the first end thereof.

Ideally, the first end of the shielding sleeve is returned inwardly within the coupling ring to define with the portion of the shielding sleeve adjacent the first end thereof an annular recess within which the coupling ring is located.

In another embodiment of the invention a diverging collar extends from and diverges from the coupling ring into the instrument accommodating lumen of the sleeve.

Preferably, the coupling ring extends externally around the instrument port of the one of the face mask and the trocar or the port connector with the first end of the shielding sleeve sealably clamped between the coupling ring and the instrument port of the one of the face mask and the trocar or the port connector.

Advantageously, the sleeve storing housing is adapted to releasably engage the one of the face mask and the trocar.

In one embodiment of the invention the closure member of the sleeve storing housing is adapted to releasably engage the one of the face mask and the trocar.

In another embodiment of the invention the one of the sleeve storing housing and the closure member thereof is adapted to releasably engage the one of the face mask and the trocar with the instrument accommodating bore of the sleeve storing housing aligned with an instrument bore extending through the trocar.

In another embodiment of the invention the one of the sleeve storing housing and the closure member thereof is adapted to releasably engage the one of the face mask and the trocar with the accommodating bore of the sleeve storing housing aligned with the instrument bore of the trocar.

In another embodiment of the invention the first end of the shielding sleeve is secured to the sleeve storing housing, and the first coupling element is adapted to couple the sleeve storing housing to the one of the face mask and the trocar.

In a further embodiment of the invention the first coupling element is adapted to non-releasably couple the sleeve storing housing to the one of the face mask and the trocar.

In another embodiment of the invention the first coupling element is configured as an engagement port for engaging an instrument port in the one of the face mask and the trocar, or a port connector, the port connector being adapted for engaging the instrument port of the one of the face mask and the trocar.

In another embodiment of the invention the shielding sleeve is stored in the storage chamber with the second end thereof extending outwardly from the storage chamber through the access opening to the second coupling element.

In another embodiment of the invention the second coupling element comprises a band adapted to extend around the instrument to releasably and sealably couple the second end of the shielding sleeve to the instrument.

Preferably, the band of the second coupling element comprises an elastic material for gripping the instrument.

Advantageously, the shielding sleeve comprises a flexible material.

Advantageously, the shielding sleeve comprises a material impermeable to air and insufflating gases.

Preferably, the shielding sleeve comprises a film material.

Advantageously, the shielding sleeve comprises a plastics material.

Further, the invention provides a face mask comprising the instrument shielding device according to the invention.

In one embodiment of the invention the instrument shielding device is coupled to the face mask by the first coupling element of the instrument shielding device.

Preferably, the first coupling element of the instrument shielding device sealably couples the instrument shielding device to the face mask.

In another embodiment of the invention the first coupling element of the instrument shielding device is releasably coupled to the face mask.

In another embodiment of the invention the instrument shielding device is non-releasably coupled to the face mask.

In another embodiment of the invention the sleeve storing housing is releasably engageable with the face mask with the instrument accommodating bore of the instrument shielding device aligned with an instrument port of the face mask.

In another embodiment of the invention the sleeve storing housing of the instrument shielding device is non-releasably engageable with the face mask.

Preferably, the first coupling element of the instrument shielding device is coupled to the instrument port of the facemask with the instrument accommodating lumen of the shielding sleeve communicating with the instrument port of the face mask.

In another embodiment of the invention an annular receiving groove extends around the instrument port of the face mask, and the first end of the shielding sleeve is secured in the instrument receiving groove by the first coupling element.

In another embodiment of the invention a collector is located in or on the face mask for collecting any air and gases escaping from the face mask around the outer periphery thereof between the face mask and the face of the subject.

In another embodiment of the invention the collector is mounted externally on the face mask.

Additionally, the invention provides a trocar comprising the instrument shielding device according to the invention.

In one embodiment of the invention the first end of the shielding sleeve of the instrument shielding device is coupled to the trocar by the first coupling element.

In another embodiment of the invention the first end of the shielding sleeve of the instrument shielding device is sealably coupled to the trocar by the first coupling element.

In another embodiment of the invention the first end of the shielding sleeve of the instrument shielding device is releasably coupled to the trocar by the first coupling element.

In a further embodiment of the invention the first end of the shielding sleeve is non-releasably coupled to the trocar by the first coupling element.

In another embodiment of the invention the first end of the shielding sleeve of the instrument shielding device is coupled directly to the trocar.

In another embodiment of the invention the sleeve storing housing is releasably engageable with the trocar with the instrument accommodating bore of the sleeve storing housing aligned with an instrument bore of the trocar.

In another embodiment of the invention the sleeve storing housing is non-releasably engageable with the trocar.

In another embodiment of the invention the first end of the shielding sleeve is coupled to one of an instrument port of the trocar and a port connector engageable with the instrument port of the trocar with the instrument accommodating lumen of the shielding sleeve communicating with the instrument port of the trocar.

Preferably, an annular receiving groove extends around the one of the instrument port of the trocar and the port connector, and the first end of the shielding sleeve is engaged in the annular receiving groove by the first coupling element.

The advantages of the invention are many. The provision of the adapter for engaging an instrument port in a face mask provides a particularly important advantage, in that gases, including gases exhaled by a subject and insufflating gases which may escape orally or nasally from the subject are filtered prior to entering the environment, and thus, any pathogens, bacteria, infections, viruses and other particular matter which may be entrained in exhaled gases and insufflating gases passing orally or nasally through the subject are filtered out of such gases prior to the gases being dispersed into the environment, and thus, the gases dispersed into the environment are clean and filtered, and are therefore safely dispersed into the environment. The instrument shielding device according to the invention provides a particularly important advantage, in that instruments being entered into a subject either orally or nasally through a face mask, or entered into a subject through a trocar or other access
device are shielded by the shielding sleeve of the instrument shielding device from the proximal end of the instrument until the instrument enters the face mask or the trocar. This, thus, protects a surgeon or other personnel handling the instrument during the procedure from being contaminated with pathogens, bacteria, infections and viruses which may reside in a cavity of a subject in which a procedure is being carried out, and which may contaminate the instrument. This, thus, prevents any danger of a surgeon or other personnel handling the instrument coming in contact with such pathogens, bacteria, infections or viruses.

The provision of a face mask with the instrument shielding device insures firstly, that no gases either exhaled gases or insufflating gases leaking orally or nasally from a subject enter the environment without first being filtered to remove any pathogens, bacteria, infections and viruses therefrom. Additionally, there is no danger of a surgeon or other personnel handling instruments which are used in the carrying out of the procedure on a subject being contaminated by pathogens, bacteria, infections and viruses which may reside in a subject, and which may contaminate the instrument.

A further advantage of the invention is achieved when the face mask is provided with a collector for collecting any gases exhaled or expelled orally or nasally from the subject, since any such gases which might escape around the periphery of the face mask between the face mask and the face of the subject are collected by the collector and subsequently filtered before being safely dispersed into the environment.

The advantages achieved when the instrument shielding device is used in conjunction with a trocar are similar to those achieved when the instrument shielding apparatus or the instrument shielding device are used in conjunction with a face mask, in that any contamination of the hands of the surgeon gripping an instrument being entered through the trocar is avoided, since the instrument is shielded by the shielding sleeve of the instrument shielding device.

Accordingly, surgeons and other healthcare professionals participating in a procedure either being carried out orally or nasally in a subject, or through a trocar, are protected from pathogens, including bacteria, infections and viruses and other particulate matter which may be exhaled by a subject or entrained in insufflating and other gases leaking from the subject, and such surgeons and other healthcare professionals are also protected from any pathogen contamination of instruments being used in the procedure.

The invention will be more clearly understood from the following description of some preferred embodiments thereof which are given by way of example only with reference to the accompanying drawings, in which:
Fig. 1 is a perspective view of a face mask comprising an adapter,
Fig. 2 is an end elevational view of the face mask of Fig. 1,
Fig. 3 is a perspective view of the face mask of Fig. 1 illustrated in use,
Fig. 4 is a perspective view of a part of the adapter of Fig. 1,
Fig. 5 is a perspective view of another portion of the adapter of Fig. 1,
Fig. 6 is a cross-sectional end elevational view of the part of Fig. 4 of the adapter of Fig. 1,
Fig. 7 is a perspective view of a face mask comprising an adapter,
Fig. 8 is a perspective view of a face mask comprising an adapter,
Fig. 9 is an end elevational view of the face mask of Fig. 8,
Fig. 10 is a perspective view of the face mask of Fig. 8 in use,
Fig. 11 is a perspective view of a face mask according to the disclosure comprising an instrument shielding apparatus according to the invention and an instrument shielding device also according to the invention for shielding an instrument, and an adapter also according to the invention coupling the instrument shielding device to the face mask,
Fig. 12 is a perspective view of the instrument shielding apparatus with the instrument shielding device of Fig. 11 in a different state to that of Fig. 11,
Fig. 13 is a perspective view of the instrument shielding device in the state of Fig. 12,
Fig. 14 is a perspective view of the instrument shielding apparatus of Fig. 11 illustrated in use,
Fig. 15 is a perspective view of the instrument shielding apparatus of Fig. 11 in use with the instrument shielding device in the state of Fig. 13,
Fig. 16 is a perspective view of the instrument shielding apparatus of Fig. 11 in use with the instrument shielding device in a state between that of Fig. 15 and Fig. 11,
Fig. 17 is another perspective view of the instrument shielding apparatus of Fig. 11 in use with the instrument shielding device in the state of Fig. 11,
Fig. 18 is a cross-sectional side elevational view of a portion of the instrument shielding device of the instrument shielding apparatus of Fig. 11,
Fig. 19 is a perspective view of a face mask according to another embodiment of the disclosure comprising an instrument shielding apparatus according to another embodiment of the invention comprising an instrument shielding device also according to another embodiment of the invention in use coupled to the face mask by an adapter according to the disclosure,
Fig. 20 is a cross-sectional side elevational view of the face mask and the instrument shielding apparatus of Fig. 19 also in use,
Fig. 21 is a perspective view of the instrument shielding apparatus of Fig. 19 illustrated in use,
Fig. 22 is a view similar to Fig. 21 illustrating the instrument shielding apparatus of Fig. 19 in another state in use,
Fig. 23 is a view similar to Fig. 21 of the instrument shielding device of the instrument shielding apparatus of Fig. 19 in another state in use,
Fig. 24 is an enlarged cross-sectional side elevational view of the instrument shielding apparatus of Fig. 19 in the state of Fig. 21,
Fig. 25 is an exploded perspective view of the instrument shielding device of the instrument shielding apparatus of Fig. 19,
Fig. 26 is a cross-sectional side elevational view of the instrument shielding apparatus of Fig. 19 in use,
Fig. 27 is a cross-sectional side elevational view of the face mask and the instrument shielding apparatus of Fig. 19 also in use,
Fig. 28 is a perspective view of a part of the instrument shielding device of the instrument shielding apparatus of Fig. 19,
Fig. 29 is a perspective view of a face mask comprising breathing apparatus,
Fig. 30 is an end elevational view of the face mask of Fig. 29,
Fig. 31 is a perspective view of the face mask of Fig. 29 in use,
Fig. 32 is a partly cross-sectional end elevational view of the breathing apparatus of Fig. 29,
Fig. 33 is a perspective view of a portion of a non-return valve for use with the breathing apparatus of Figs. 29 to 32, and which may be used in any of the adapters described herein,
Fig. 34 is an exploded perspective view of the non-return valve of Fig. 33,
Fig. 35(a) and (b) are diagrammatic side elevational views of the non-return valve of Fig. 33 in two different states of use,
Fig. 36 is a partly cross-sectional end elevational view of a face mask,
Fig. 37 is a perspective view of another face mask,
Fig. 38 is a perspective view of the face mask of Fig. 37 in use,
Fig. 39 is a perspective view of a further face mask,
Fig. 40 is an end elevational view of the face mask and the adapter of Fig. 39,
Fig. 41 is a perspective view of the face mask and the adapter of Fig. 39 in use,
Fig. 42 is a partly cross-sectional side elevational view of the adapter of Fig. 39,
Fig. 43 is a perspective view of a face mask according to another embodiment of the disclosure comprising an instrument shielding apparatus also according to the invention, the instrument shielding apparatus comprising an instrument shielding device also according to the invention and an adapter
Fig. 44 is a perspective view of the instrument shielding apparatus of Fig. 43,
Fig. 45 is an end elevational view of the instrument shielding apparatus of Fig. 43 in a different state to that of Fig. 44,
Fig. 46 is a partly cross-sectional end elevational view of the instrument shielding apparatus of Fig. 43 in the state of Fig. 45,
Fig. 47 is a partly cross-sectional exploded end elevational view of a portion of the instrument shielding apparatus of Fig. 43,
Fig. 48 is a perspective view of the instrument shielding apparatus of Fig. 43 in use,
Fig. 49 is another perspective view of the instrument shielding apparatus of Fig. 43 also in use,
Fig. 50 is a perspective view of the instrument shielding apparatus of Fig. 43 also in use but in the state of Fig. 44,
Fig. 51 is a perspective view of an instrument shielding apparatus according to another embodiment of the invention,
Fig. 52 is an end elevational view of the instrument shielding apparatus of Fig. 51,
Fig. 53 is an end elevational view of the instrument shielding apparatus of Fig. 51 in a different state to that of Fig. 51,
Fig. 54 is a cross-sectional end elevational view of the instrument shielding apparatus of Fig. 51 in the state of Fig. 51,
Fig. 55 is an exploded perspective view of the instrument shielding apparatus of Fig. 51,
Fig. 56 is another exploded perspective view of the instrument shielding apparatus of Fig. 51,
Fig. 57 is an exploded end elevational view of the instrument shielding apparatus of Fig. 51,
Fig. 58 is another perspective view of the instrument shielding apparatus of Fig. 51,
Fig. 59 is a further perspective view of the instrument shielding apparatus of Fig. 51,
Fig. 60 is a perspective view of an adapter of the instrument shielding apparatus of Fig. 51,
Fig. 61 is a perspective view of a portion of an instrument shielding device of the instrument shielding apparatus of Fig. 51,
Fig. 62 is a cross-sectional side elevational view of a detail of the instrument shielding apparatus of Fig. 51,
Fig. 63 is another enlarged cross-sectional side elevational view of a detail of the instrument shielding apparatus of Fig. 51,
Fig. 64 is a side elevational view of a trocar comprising the instrument shielding apparatus of Figs. 51 to 63 engaged therein,
Fig. 65 is a side elevational view of the trocar of Fig. 64 with the instrument shielding apparatus of Figs. 51 to 63 engaged therein in a different state to that of Fig. 64, and
Fig. 66 is a cross-sectional end elevational view similar to that of Fig. 54 of an instrument shielding apparatus according to another embodiment of the invention.

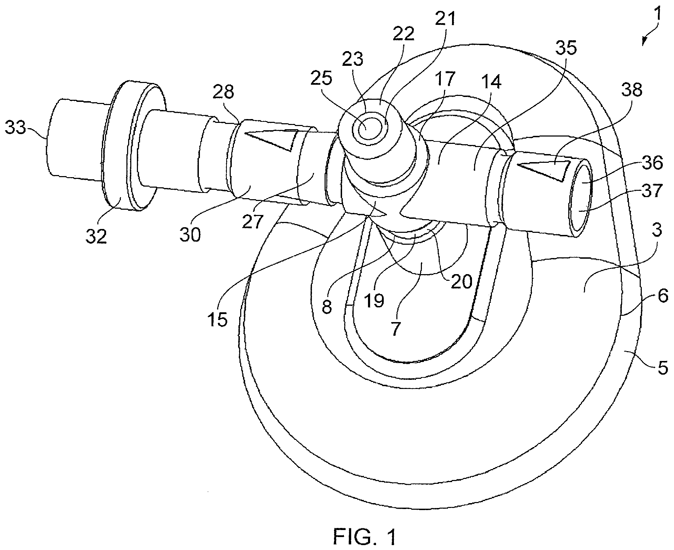

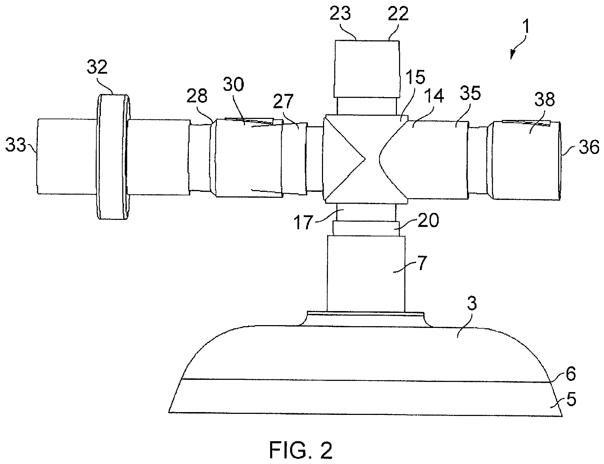

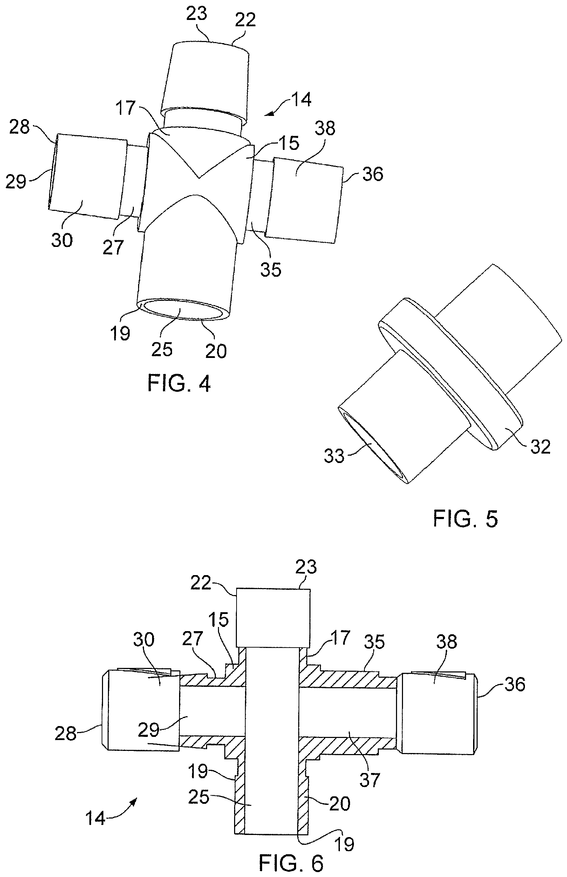

Referring to the drawings and initially to Figs. 1 to 6 thereof, there is illustrated a face mask indicated generally by the reference numeral 1 for securing to the face of a subject during, for example, an endoscopic procedure. The face mask 1 is sized and shaped to cover the mouth and nose of the face of the subject, and to extend around the chin of the subject, upwardly over the cheeks of the subject, and across the bridge of the nose of the subject.

The face mask 1 comprises a central portion 3, and an outer peripheral tubular portion 5 extending around a peripheral edge 6 of the central portion 3, and sealably secured to the central portion 3. The outer peripheral portion 5 comprises a flexible sealed tube which may be pre-air filled or configured to be pumped with air by, for example, a syringe or other suitable pump. The tube when air filled forms a peripheral cushion extending around the central portion 3 for forming a substantially air tight seal with the face of the subject between the face mask 1 and the face of the subject. A port, in this case an instrument port 7 extends from the central portion 3 of the face mask 1, and defines a passageway 8 extending therethrough to provide access to the mouth or nose of the subject, and in this case, to accommodate an instrument, for example, an endoscope 10 through the face mask 1 for entry orally or nasally into the subject.

An adapter indicated generally by the reference numeral 14 is engaged in the instrument port 7 of the face mask 1 for accommodating the endoscope 10 through the face mask 1 for oral or nasal entry into the subject, and also for accommodating breathing by the subject.

The adapter 14 comprises an instrument accommodating housing, namely, a housing 15 which is formed by an elongated main tubular member 17 extending between a first end 19 and a second end 22. The main tubular member 17 terminates at the first end 19 in a first main port 20 configured to sealably and releasably engage the instrument port 7 of the face mask 1. The main tubular member 17 terminates at the second end 22 in a second main port 23. An instrument accommodating bore, namely, an instrument bore 25 extends through the main tubular member 17 between and communicates with the first and second main ports 20 and 23 for accommodating the endoscope 10 therethrough, and in turn through the face mask 1 to the subject. A suitable sealing means is located in the second main port 23 or in the instrument bore 25 to both sealably and slideably engage the endoscope 10 as it is being manoeuvred inwardly and outwardly of the subject during the procedure in order to prevent leakage of air and other gases exhaled or otherwise expelled orally or nasally by or from the subject. The sealing means is illustrated in Fig. 1 as an annular seal 21 located in the second main port 23. However, it is envisaged that the sealing means may, for example, be provided in the form of a duckbill valve, a multi-leaf valve, a cross-slit valve or other suitable valve or sealing means located in the second main port 23 or in the instrument bore 25.

A first tubular member 27 extends from the main tubular member 17 intermediate the first end 19 and the second end 22 thereof, and terminates in a first air accommodating port, namely, a first secondary port 28. A first bore 29 extends through the first tubular member 27 from the first secondary port 28 and terminates in and communicates with the instrument bore 25. The first bore 29 extends from the instrument bore 25 at a location intermediate the annular seal 21 and the first main port 20. A first non-return valve 30 is located in the first tubular member 27 intermediate the first secondary port 28 and the main tubular member 17. The first non-return valve 30 defines the first secondary port 28, although the first non-return valve 30 may be independent of the first secondary port 28. The first non-return valve 30 is adapted to permit fluid flow through the first bore 29 in an outward direction from the instrument bore 25 to the first secondary port 28 in order to accommodate air and gases expelled by the subject, and other gases, for example, insufflating gases, which if a cavity or vessel in which the procedure is being carried out is being insufflated may leak through the mouth or nose of the subject. The first non-return valve 30 is adapted to prevent fluid flow in an inward direction from the first secondary port 28 to the instrument bore 25.

The adapter 14 also comprises a first filter 32 which is coupled to the first secondary port 28 for accommodating air and gases from the first secondary port 28 therethrough, and for filtering out particulate matter, including pathogens, such as bacteria, viruses and infections from the air and gases passing therethrough. The first filter comprises a blended synthetic fibre material housed in a polypropylene housing and is configured to filter out particulate matter of size greater than 0.05 microns. Filtered air and gases are safely dispersed from the first filter 32 into the environment through a discharge port 33 extending from the first filter 32.

A second tubular member 35 extends from the main tubular member 17 intermediate the first end 19 and the annular seal 21 on the opposite side of the main tubular member 17 to that from which the first tubular member 27 extends. The second tubular member 35 terminates in a second secondary port 36. A second bore 37 extends from the second secondary port 36 to the instrument bore 25 and communicates with the instrument bore 25 for accommodating air or any other gas therethrough for inhaling or to be inhaled by the subject. A second non-return valve 38 is located in the second bore 37 between the second secondary port 36 and the instrument bore 25. The second non-return valve 38 defines the second secondary port 36, although the second non-return valve 38 may be provided to be independent of the second non-return valve 38. The second non-return valve 38 is adapted to permit fluid flow, namely, air and any other cases to be inhaled by the subject to flow in an inward direction from the second secondary port 36 to the instrument bore 25, and in turn through the instrument bore 25 to the subject. The second non-return valve 38 is adapted to prevent fluid flow through the second bore 37 in an outward direction from the instrument bore 25 to the second secondary port 36.

The diameter of the instrument bore 25 is greater than the diameter of the endoscope 10 in order to accommodate air and gases to and from the subject therethrough.

In use, with the first filter 32 secured to the first secondary port 28 of the adapter 14, the adapter 14 is engaged with the face mask 1 by engaging the first main port 20 in the instrument port 7 of the face mask 1. The face mask 1 is then secured to the face of the subject in a conventional manner, by, for example, loops (not shown) extending from the central portion 3 adjacent the peripheral edge 6 thereof engaging the ears of the subject, or by bands extending from the face mask 1 adjacent the peripheral edge 6 around the head of the subject. With the face mask 1 so secured to the subject, the subject can breathe through the adapter 14 with inhaled air being drawn through the adapter 14 from the second secondary port 36, and air and other gases being expelled by the subject being expelled through the first secondary port 28, and in turn through the first filter 32.

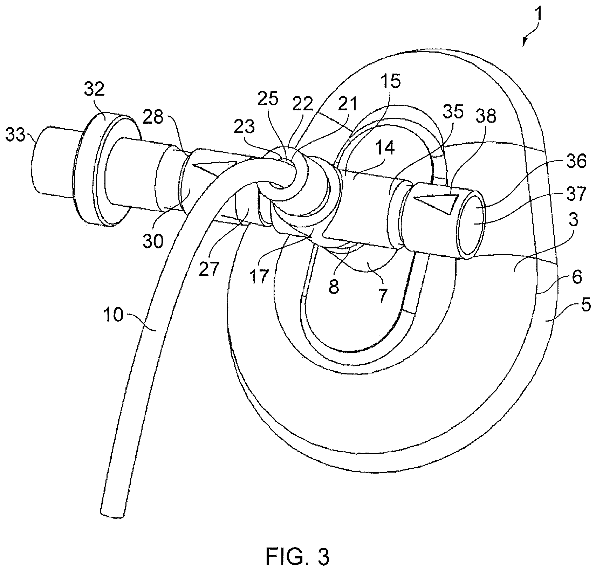

The endoscope 10 as illustrated in Fig. 3 is then entered into the instrument bore 25 through the second main port 23 of the housing 15 and in turn through the face mask 1 and then orally or nasally into the subject.

The surgeon is then free to manoeuvre the endoscope 10 through the adapter 14 to carry out the procedure. While the procedure is being carried out, the subject breathes by inhaling air through the second secondary port 36 and exhaling through the first secondary port 28, and in turn through the first filter 32. Thus, any pathogens and other particulate matter entrained in the exhaled air and other gases exhaled by or expelled orally or nasally from the subject are retained by the first filter 32, and the exhaled air and gases are safely delivered and dispersed into the environment through the discharge port 33 of the first filter 32.

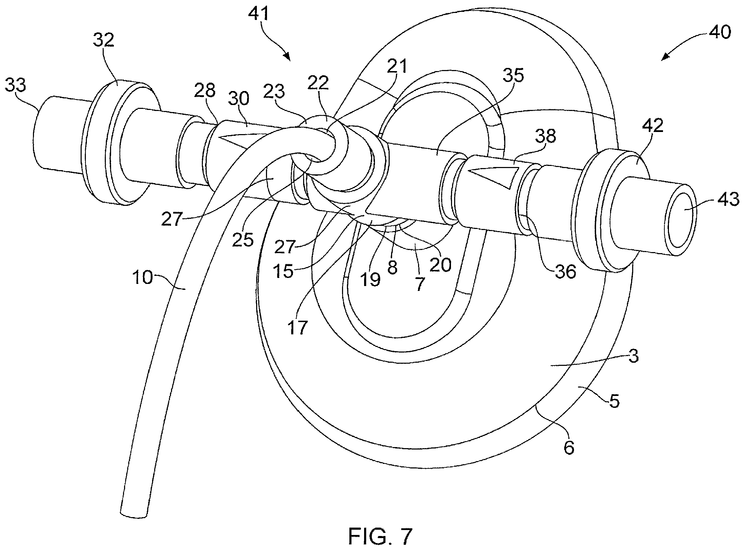

Referring now to Fig. 7 there is illustrated a face mask indicated generally by the reference numeral 40 for securing to the face of a subject during a surgical or investigative procedure, typically, which would be carried out by orally or nasally entering an instrument, for example, an endoscope 10, into a subject to a site in which the procedure is to be carried out. The face mask 40 is substantially similar to the face mask 1 described with reference to Figs. 1 to 6, and similar components are identified by the same reference numerals. An adapter indicated generally by the reference numeral 41 is releasably and sealably secured to the face mask 40 for sealably accommodating the instrument or endoscope 10 therethrough to the mouth or nose of the subject. The adapter 41 is substantially similar to the adapter 14 described with reference to Figs. 1 to 6, and similar components are identified by the same reference numerals.

The only difference between the face mask 40 and the face mask 1, and the adapter 41 and the adapter 14 is that the adapter 41 comprises a second filter, namely, a second filter 42 coupled to the second secondary port 36 for filtering air for inhaling by the subject, as the air is being drawn therethrough, and in turn through the second non-return valve 38, the second bore 37 and the instrument bore 25 of the housing 15 of the adapter 14. An inlet port 43 to the second filter 42 accommodates air for inhaling by the subject into the second filter 42, and in turn through the second non-return valve 38, the second bore 37 and the instrument bore 25 to the subject.

The second filter 42 is similar to the first filter 32, and is adapted to filter out particles of size greater than 0.05 microns from the air to be inhaled by the subject. Accordingly, the second filter 42 minimises the risk of air being inhaled by the subject containing pathogens, bacteria, infections and viruses. However, it will be appreciated that the second filter may not be similar to the first filter and may be a coarser or a finer filter than the first filter.

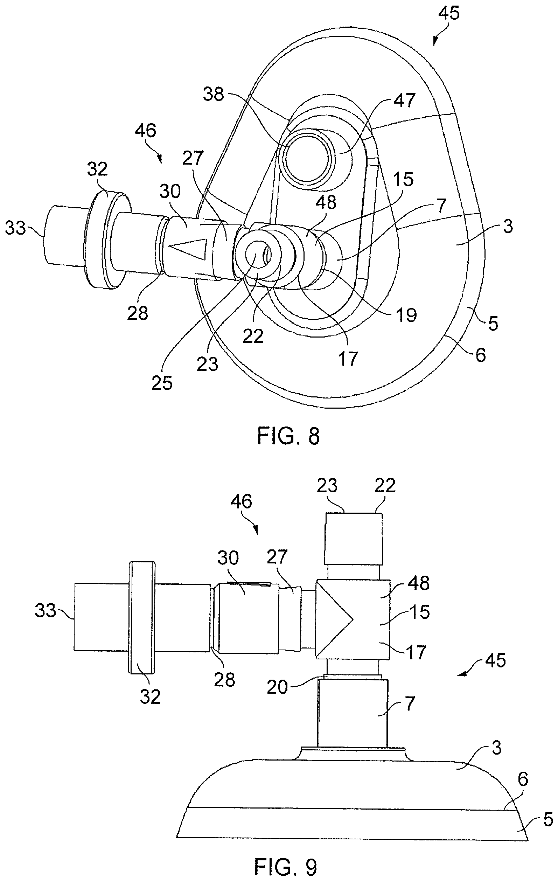

Referring now to Figs. 8 to 10 there is illustrated a face mask indicated generally by the reference numeral 45 also for securing to the face of a subject for accommodating an instrument, for example, an endoscope therethrough for oral or nasal entry into the subject for carrying out a procedure at a site internally in the subject. The face mask 45 comprises an adapter indicated generally by the reference numeral 46. The face mask 45 is substantially similar to the face mask 1, and similar components are identified by the same reference numerals. The main difference between the face mask 45 and the face mask 1 is that as well as comprising an instrument port 7, the face mask 45 also comprises a secondary port 47, which typically is an air accommodating port for accommodating air and other gases to be inhaled by a subject or expelled by the subject.

Turning now to the adapter 46, the adapter 46 is substantially similar to the adapter 14 described with reference to Figs. 1 to 6, and similar components are identified by the same reference numerals. The housing 15 of the adapter 46 is provided without the second tubular member 35, but is provided with the first tubular member 27, the first non-return valve 30 and the first secondary port 28. The first non-return valve 30 is configured in a similar manner as the first non-return valve 30 of the adapter 14, and is adapted to permit fluid flow in the outward direction through the first bore 29 from the instrument bore 25 to the first secondary port 28. Accordingly, air and other gases exhaled by or otherwise expelled orally or nasally from the subject are accommodated through the adapter 46, as well as any other gases, for example, insufflating gases which leak orally or nasally from the subject. All such air and gases are accommodated through the first bore 29 and in turn through the first filter 32, through which filtered air and gases are safely dispersed into the atmosphere through the discharge outlet port 33 of the first filter 32.

The secondary port 47 in the face mask 45 is used to accommodate air for inhaling by the subject. In order to avoid any danger of exhaled air or gases or leaked insufflating gases passing into the environment through the secondary port 47, the second non-return valve 38 is sealably engaged in the secondary port 47 of the face mask 45. The second non-return valve 38 is adapted to permit fluid flow in an inward direction through the secondary port 47 and into the face mask 45 in order to accommodate air and other gases to the subject to be inhaled by the subject. The second non-return valve 38 is adapted to prevent fluid flow in an outward direction from the face mask 45 through the secondary port 47 thereof, in order to prevent air and other gases expelled by the subject passing into the environment through the secondary port 47 from the face mask 45.

Otherwise, the face mask 45 and the adapter 46 are similar to the face mask 1 and the adapter 14 described with reference to Figs. 1 to 6 and their use is similar to that described with reference to the face mask 1.

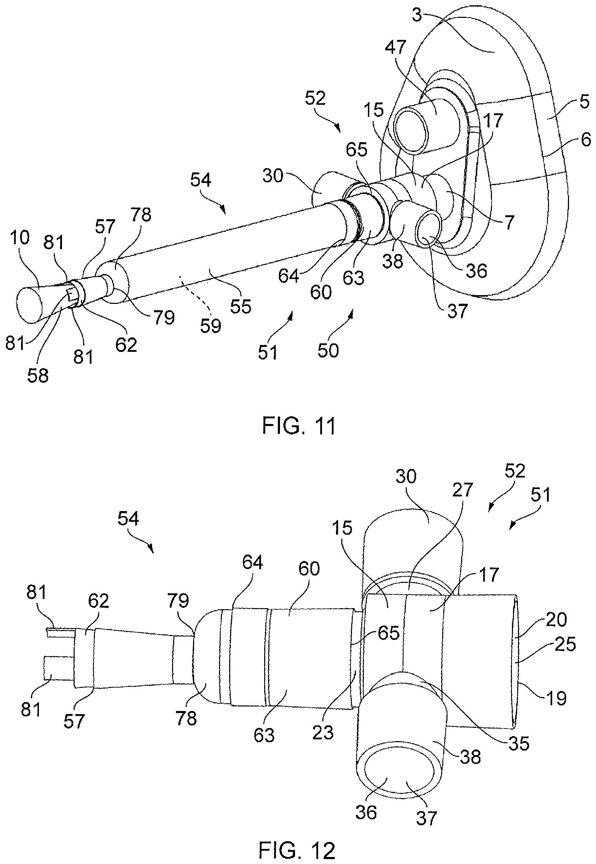

Referring now to Figs. 11 to 18, there is illustrated a face mask according to another embodiment of the disclosure indicated generally by the reference numeral 50 for securing to the face of a subject to accommodate an instrument, for example, an endoscope 10 therethrough for oral or nasal entry into the subject. The face mask 50 comprises an instrument shielding apparatus, also according to the invention and indicated generally by the reference numeral 51. The instrument shielding apparatus 51 comprises an adapter also according to the invention and indicated generally by the reference numeral 52, and an instrument shielding device also according to the invention and indicated generally by the reference numeral 54 coupled to the adapter 52 for shielding an instrument, for example, the endoscope 10 during use thereof by a surgeon. The face mask 50 is similar to the face mask 45 described with reference to Figs. 8 to 10, and similar components are identified by the same reference numerals. The adapter 52 is similar to the adapter 14 of the face mask 1 described with reference to Figs. 1 to 6 and similar components are identified by the same reference numerals. Although not illustrated in Figs. 11 to 18, the adapter 52 is provided with a first filter 32 coupled to the first secondary port 28 defined by the first non-return valve 30, and may also be provided with a second filter similar to the second filter 42 of the adapter 41 described with reference to Fig. 7. The second filter would be coupled to the second secondary port 36 defined by the second non-return valve 38.

The adapter 52 is secured to the face mask 50 with the first main port 20 of the adapter 52 releasably and sealably engaged with the instrument port 7 of the face mask 50. The instrument shielding device 54 is releasably and sealably engaged with the second main port 23 of the housing 15 of the adapter 52 as will be described below. Although, the instrument shielding device 54 may be non-releasably secured to the second main port 23 of the housing 15 of the adapter 52, and in which case, the adapter 52 and the instrument shielding device 54 would be supplied as a single unit.

Turning now to the instrument shielding device 54, the instrument shielding device 54 comprises a sleeve storing housing 60 adapted for coupling to the second main port 23 of the adapter 52 as will be described below. An annular storage chamber 61 is formed in the sleeve storing housing 60 as will also be described below.

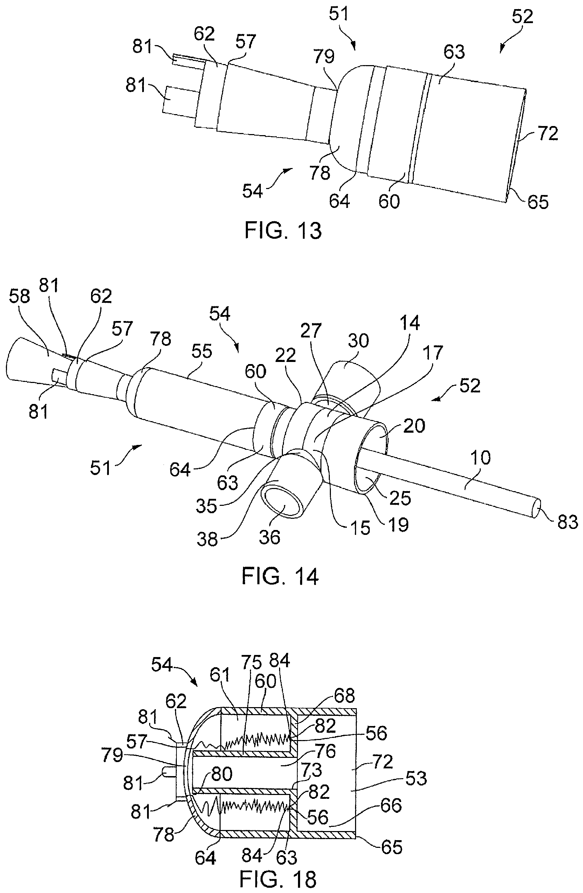

An elongated shielding sleeve 55 of a flexible material, in this embodiment of the invention an air tight flexible film material, which is impermeable to insufflating gases, and preferably is transparent, in this embodiment of the invention comprises a transparent polyurethane film material. The shielding sleeve 55 extends between a first end 56 and a second end 57 and defines an elongated instrument accommodating lumen 59 extending therethrough from the first end 56 to the second end 57 for accommodating the endoscope 10 therethrough. The first end 56 of the shielding sleeve 55 is sealably secured to the sleeve storing housing 60 in the storage chamber 61 thereof, as will be described below, and the sleeve storing housing 60 forms a first coupling element 53 for releasably and sealably coupling the sleeve storing housing 60, and in turn the first end 56 of the shielding sleeve 55 to the adapter 52 as will also be described below. The second end 57 of the shielding sleeve 55 terminates in a second coupling element, namely, an engagement band 62 and is sealably secured to the engagement band 62. The engagement band 62 is adapted to releasably and sealably engage the endoscope 10 or other instrument adjacent a proximal end 58 thereof. The engagement band 62 is of an elastic material so that it tightly contracts onto an outer proximally diverging peripheral surface of the endoscope 10 adjacent the proximal end 58 thereof. The shielding sleeve 55 is urgeable as will be described below from a collapsed stored state stored in the storage chamber 61 of the sleeve storing housing 60 as illustrated in Fig. 18, to an extended state extending out of the storage chamber 61 and shielding the endoscope 10 as illustrated in Fig. 11.

The sleeve storing housing 60 comprises a cylindrical outer side wall 63 extending between a first end 64 and a second end 65 and defining a central bore 66 extending therethrough. A circular base plate 68 is located in the central bore 66 intermediate the first and second ends 64 and 65 and divides the bore 66 into a first portion forming the storage chamber 61 within which the shielding sleeve 55 is stored in the collapsed stored state, and a second portion forming the first coupling element 53 as an engagement port 72 for releasably and sealably engaging the second main port 23 of the housing 15 of the adapter 52. A central opening 73 extends through the base plate 68 for accommodating the endoscope 10 therethrough.

A tubular member, namely, a cylindrical secondary tubular member 75 extends from the base plate 68 centrally through the storage chamber 61, and defines with the outer side wall 63 of the sleeve storing housing 60 and the base plate 68 the storage chamber 61 of annular shape. The secondary tubular member 75 defines an instrument accommodating bore, namely, a secondary instrument accommodating bore 76 extending therethrough and communicating with the engagement port 72 through the central opening 73 in the base plate 68 for accommodating the endoscope 10 therethrough. The secondary instrument accommodating bore 76 is aligned with the instrument bore 25 of the adapter 52 when the engagement port 72 is engaged with the second main port 23 of the adapter 14, for in turn accommodating the endoscope 10 into the instrument bore 25 of the adapter 52.

A closure member, in this case a dome shaped closure member 78 which is releasably secured to the outer side wall 63 of the sleeve storing housing 60 adjacent the first end 64 thereof partly closes the storage chamber 61. A central access opening 79 formed in the closure member 78 accommodates the shielding sleeve 55 therethrough from the storage chamber 61 to the engagement band 62 adjacent the closure member 78. The central access opening 79 in the closure member 78 is centrally aligned with the secondary tubular member 75 when the closure member 78 is secured to the outer side wall 63, and defines with the secondary tubular member 75 an annular access opening 80 for accommodating the shielding sleeve 55 therethrough from the storage chamber 61 to the engagement band 62.

The first end 56 of the shielding sleeve 55 defines a peripheral edge 84, and the first end 56 of the shielding sleeve 55 is sealably secured to the base plate 68 at 82 in the storage chamber 61 with the peripheral edge 84 thereof sealably secured to the base plate 68 around the secondary tubular member 75. The peripheral edge 84 of the first end 56 of the shielding sleeve 55 may be sealably secured to the base plate 68 by any suitable adhesive or mechanical clamping system. In the collapsed stored state, the shielding sleeve 55 is located within the annular storage chamber 61 with the collapsed shielding sleeve 55 extending around the secondary tubular member 75, and with the secondary tubular member 75 extending into the instrument accommodating lumen 59 of the shielding sleeve 55.

Four finger grip tabs 81 extend from the engagement band 62, and are equi-spaced apart circumferentially around the engagement band 62. The finger grip tabs 81 are provided to facilitate urging the engagement band 62 into engagement with the diverging proximal end 58 of the endoscope 10, in order to ensure that the engagement band 62 tightly and sealably engages the endoscope 10.

Since in this embodiment of the invention the secondary port 47 of the face mask 50 is not required, it is sealably closed by a suitable releasable closure plug (not shown). However, if the secondary port 47 were required to accommodate another instrument or an insufflating tube orally or nasally into the subject, a suitable sealing means would be located in the secondary port 47 for slideably and sealably engaging the instrument or insufflating tube in the secondary port 47.

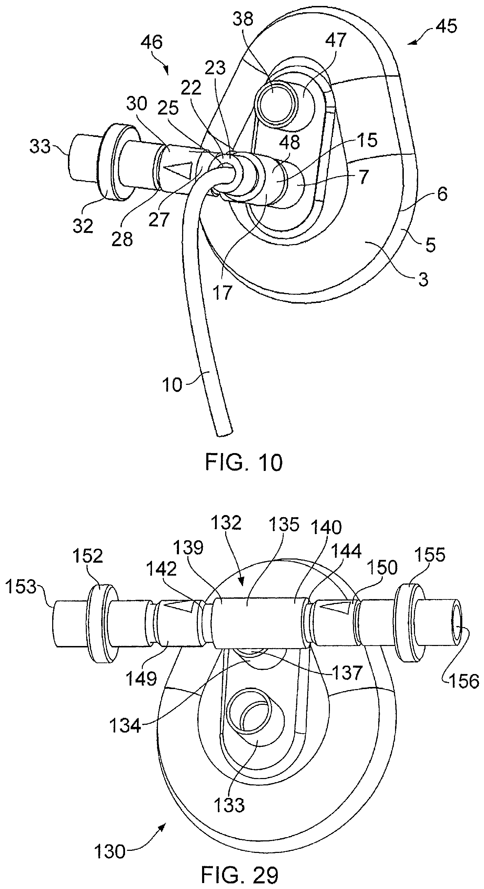

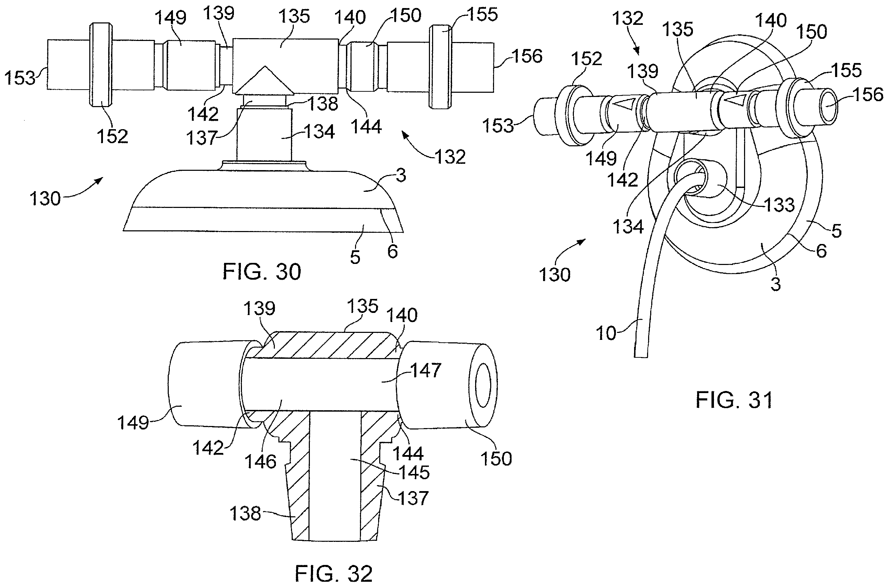

It will of course be appreciated that in another embodiment of the invention the instrument shielding device 54 instead of being connected to the face mask 50 through the adapter 52 could be directly coupled to the face mask 50 by suitably adapting the engagement port 72 of the instrument shielding device 54 for directly engaging the main port 7 of the face mask 50. in which case, the breathing apparatus which is described below with reference to Figs. 29 to 32, and is indicated generally by the reference numeral 132 would be engaged in the secondary port 47 of the face mask 50 in order to facilitate breathing by the subject through the secondary port 47 of the face mask 50 and in turn through the breathing apparatus 132. Alternatively, a filter similar to the first or second filters may be sealably engaged in the secondary port 47 to filter air and other gases being inhaled and exhaled or otherwise expelled orally or nasally by the subject.

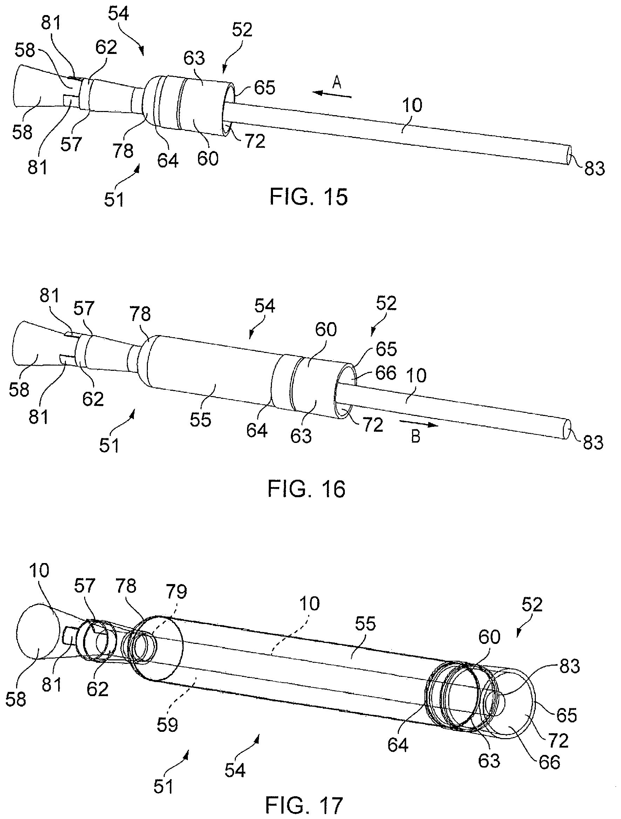

In use, with the adapter 52 engaged in the instrument port 7 of the face mask 50, the face mask 50 is secured to the face of the subject as already described with reference to the face mask 1. With the shielding sleeve 55 in the collapsed stored state in the sleeve storing housing 60, and the sleeve storing housing 60 disengaged from the adapter 14, the distal end 83 of the endoscope 10 is entered through the engagement band 62, and in turn through the second end 57 of the shielding sleeve 55 and through the central access opening 79 in the closure member 78, and then through the secondary instrument accommodating bore 76 of the instrument shielding device 54. The instrument shielding device 54 is urged proximally along the endoscope 10 in the direction of the arrow A until the engagement band 62 tightly and sealably engages the diverging proximal end 58 of the endoscope 10, see Fig. 15. The finger grip tabs 81 are used to urge the engagement band 62, and in turn the second end 57 of the shielding sleeve 55 into tight sealing engagement with the diverging proximal end 58 of the endoscope 10.

With the engagement band 62 and the second end 57 of the shielding sleeve 55 tightly engaged with the endoscope 10, the sleeve storing housing 60 is disengaged from the closure member 78, and is then urged distally along the endoscope 10 in the direction of the arrow B, thereby urging the shielding sleeve 55 from the stored state in the storage chamber 61 into the extended state to protect and shield the endoscope 10, see Figs. 16 and 17. The distal end 83 of the endoscope 10 is then entered into the instrument accommodating bore 25 in the adapter 52 through the second main port 23. The sleeve storing housing 60 of the instrument shielding device 54 is engaged with the adapter 52 by engaging the engagement port 72 of the sleeve storing housing 60 with the second main port 23 of the adapter 52, with the secondary instrument accommodating bore 76 of the sleeve storing housing 60 aligned with the instrument bore 25 of the adapter 52.

The endoscope 10 is gripped through the shielding sleeve 55, and the endoscope 10 is then urged through the instrument bore 25 of the adapter 52, and through the instrument port 7 of the face mask 50, and in turn orally or nasally into the subject.

Thereafter, the surgeon manipulates the endoscope 10 by gripping the endoscope 10 through the shielding sleeve 55. The subject breathes through the adapter 52, with inhaled air drawn through the second secondary port 36, and exhaled air and other gases filtered through the first filter. The clean filtered air and gases from the first filter are safely dispersed into the environment.

On completion of the procedure, the endoscope 10 is withdrawn from the subject, and the shielding sleeve 55 extends further from the storage chamber 61. When the shielding sleeve 55 of the instrument shielding device 54 has been fully extended, the engagement port 72 of the sleeve storing housing 60 is disengaged from the second main port 23, and the remainder of the endoscope is withdrawn through the instrument bore 25 of the adapter 52. Thereafter, the face mask 50 is removed from the face of the subject, and the endoscope 10 is withdrawn from the instrument shielding device 54.

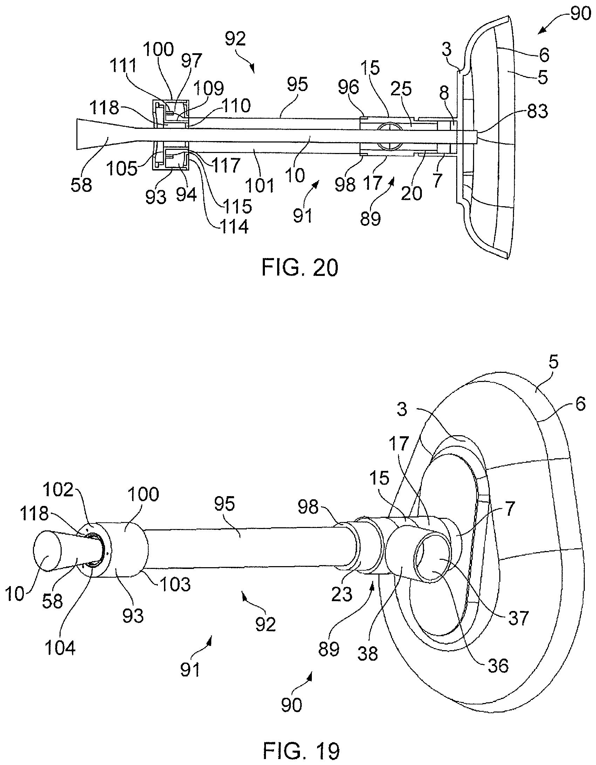

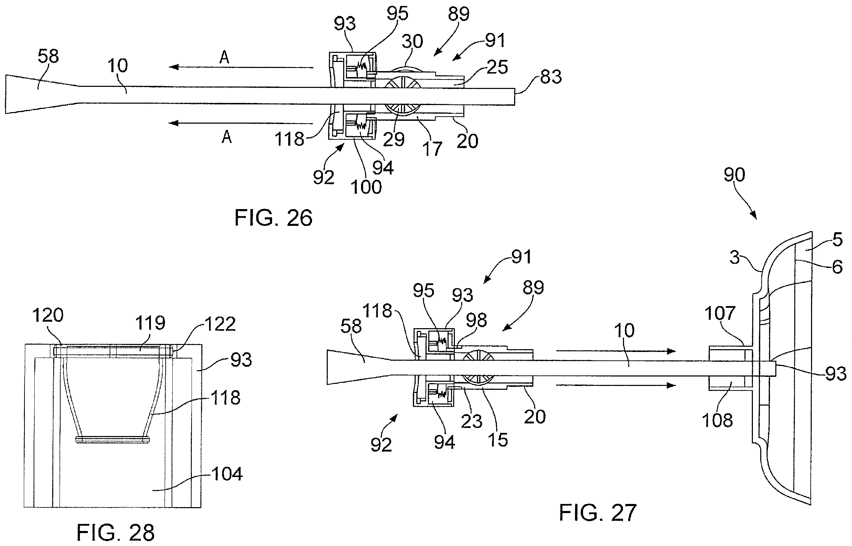

Referring now to Figs. 19 to 28, there is illustrated a face mask according to another embodiment of the disclosure indicated generally by the reference numeral 90 for securing to the face of a subject to accommodate an instrument, for example, an endoscope 10 therethrough for oral or nasal entry into the subject in order to carry out a procedure in a vessel, cavity or lumen within the subject. The face mask 90 is similar to the face mask 1 described with reference to Figs. 1 to 6, and similar components are identified by the same reference numerals.

The face mask 90 comprises an instrument shielding apparatus, also according to the invention and indicated generally by the reference numeral 91. The instrument shielding apparatus 91 comprises an adapter also according to the invention and indicated generally by the reference numeral 89, and an instrument shielding device also according to the invention and indicated generally by the reference numeral 92. The adapter 89 is substantially similar to the adapter 14 of the face mask 1 described with reference to Fig. 1 to 6, and similar components are identified by the same reference numerals. Although not illustrated in the drawings, the adapter 89 is provided with a first filter, which is similar to the first filter of the adapter 14, and which is coupled to the first secondary port 28 defined by the first non-return valve 30 for filtering out pathogens, bacteria, infections and viruses in air and other gases exhaled by or expelled orally or nasally from the subject. The adapter 89 may also be provided with a second filter, similar to the second filter 42 of the adapter 41 described with reference to Fig. 7, which would filter out pathogens, bacteria, infections and viruses in air being inhaled by the subject. The instrument shielding device 92 is coupled to the adapter 89 for shielding an instrument, for example, the endoscope 10 being entered orally or nasally into a subject through the adapter 89 and in turn through the face mask 90.

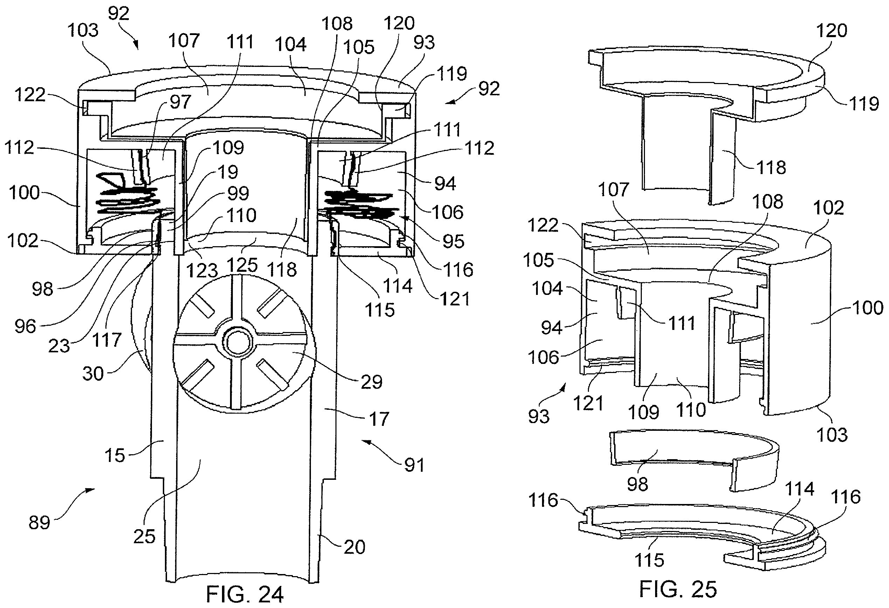

In this embodiment of the invention the instrument shielding device 92 comprises a sleeve storing housing 93. The sleeve storing housing 93 defines an annular storage chamber 94 within which an instrument shielding sleeve 95 is stored in a collapsed stored state illustrated in Fig. 24. The shielding sleeve 95 is substantially similar to the shielding sleeve 55 of the instrument shielding device 54 of the face mask 50 described with reference to Figs. 11 to 18, and defines an instrument accommodating lumen 101 extending therethrough for accommodating an instrument, for example, the endoscope 10 for shielding thereof.

The shielding sleeve 95 extends between a first end 96 and a second end 97, and is urgeable from the collapsed stored state located within the storage chamber 94 illustrated in Fig. 24 to an extended state illustrated in Figs. 19 and 20 shielding the endoscope 10. The first end 96 of the shielding sleeve 95 is sealably secured to the second main port 23 of the housing 15 of the adapter 89 by a first coupling element which in this embodiment of the invention comprises a coupling ring 98. The coupling ring 98 extends tightly around an outer peripheral portion 99 of the second main port 23, with the first end 96 of the shielding sleeve 95 securely and sealably clamped between the coupling ring 98 and the outer peripheral portion 99 the second main port 23.

Turning now to the sleeve storing housing 93, the sleeve storing housing 93 comprises a cylindrical outer side wall 100 extending between a first end 102 and a second end 103 and defining a bore 104 extending therethrough. A base plate 105 extending transversely in the bore 104 divides the bore 104 into a first portion 106 and a second portion 107. A central opening 108 extending through the base plate 105 accommodates an instrument, for example, the endoscope 10 therethrough. A secondary tubular member 109 of circular transverse cross-section extending from the base plate 105 and around the central opening 108 extends into the first portion 106 and defines with the base plate 105 and the corresponding portion of the outer side wall 100 the annular storage chamber 94. The secondary tubular member 109 defines a secondary instrument accommodating bore 110 for accommodating the instrument or endoscope 10 therethrough into the instrument bore 25 of the adapter 89.

An intermediate cylindrical wall 111 extends from the base plate 105 into the annular storage chamber 94, and is spaced apart between and from the outer side wall 100 and the secondary tubular member 109. A clamping ring 112 which tightly fits around the intermediate cylindrical wall 111 engages and sealably clamps the second end 97 of the shielding sleeve 95 between the intermediate cylindrical wall 111 and the clamping collar 112, for in turn sealably securing the second end 97 of the shielding sleeve 95 to the base plate 105 of the sleeve storing housing 93.

A closure member 114 having a central access opening 115 closes the storage chamber 94. The closure member 114 is secured to the outer side wall 100 adjacent the first end 102 thereof by means of a snap-fit coupling ring 116 extending from the closure member 114. The snap-fit coupling ring 116 engages a circumferential flange 121 extending circumferentially around and inwardly from the outer side wall 100 of the sleeve storing housing 93 adjacent the first end 102 with a snap-fit action for securing the closure member 114 to the sleeve storing housing 93. A rim 124 defining the central access opening 115 in the closure member 114 defines with an end 125 of the secondary tubular member 109, an annular opening 117 for accommodating the sleeve 95 therethrough from the storage chamber 94.

The diameter of the central access opening 115 in the closure member 114 is such as to slideably engage the coupling ring 98, and an annular recess 123 extends into the second main port 23 for slideably engaging the secondary tubular member 109 of the sleeve storing housing 93 adjacent the end 125 thereof, so that when the shielding sleeve 95 is clamped onto the second main port 23 by the coupling ring 98 and is in the stored state in the storage chamber 94, the sleeve storing housing 93 is releasably engageable with the second main port 23 of the adapter 89. When the sleeve storing housing 93 is engaged with the second main port 23 of the adapter 89, the secondary instrument accommodating bore 110 of the sleeve storing chamber 93 is aligned with the second main port 23 and the instrument accommodating bore 25 of the adapter 89 for accommodating the instrument from the instrument shielding device 92 to the adapter 89.

A second coupling element for releasably and sealably coupling the sleeve storing housing 93 onto the endoscope 10 adjacent the proximal end 58 thereof comprises a duckbill valve 118 anchored in the second portion 107 of the sleeve storing housing 93 and extending into the secondary instrument accommodating bore 110. The duckbill valve 118 tightly, but releasably and sealably engages the instrument or endoscope 10 in the sleeve storing housing 93. The duckbill sealing valve 118 prevents the escape of air and other gases exhaled, expelled or leaking orally or nasally from the subject through the secondary instrument accommodating bore 110 past the proximal end 58 of the endoscope 10. An anchoring rim 119 extending circumferentially around and radially outwardly from an end 120 of the duckbill valve 118 is sealably anchored in an annular recess 122 extending into the cylindrical outer wall 100 from the bore 104 adjacent the second end 102 of the cylindrical outer side wall 100. Such duckbill valves as the duckbill valve 118 will be well known to those skilled in the art. It will be appreciated that any other suitable second coupling element for releasably but tightly and sealably engaging the endoscope 10 adjacent the distal end 58 thereof or other instrument may be provided, for example, a multi-leaf valve, a cross-slit valve or any other such means which would sealably engage and releasably secure the sleeve storing housing 93 on the proximal end 58 of the endoscope 10 or other instrument.

In this embodiment of the invention, because the first end 96 of the shielding sleeve 95 is clamped onto the second main port 23 of the adapter 89 by the coupling ring 98, the instrument shielding device 92 is non-releasably secured to the adapter 89, and accordingly, the instrument shielding apparatus 91 comprising the adapter 89 and the instrument shielding device 92 is supplied as a single unit with the shielding sleeve 95 in the collapsed state in the storage chamber 94 and the sleeve storing housing 93 releasably engaged on the second main port 23 of the adapter 89.

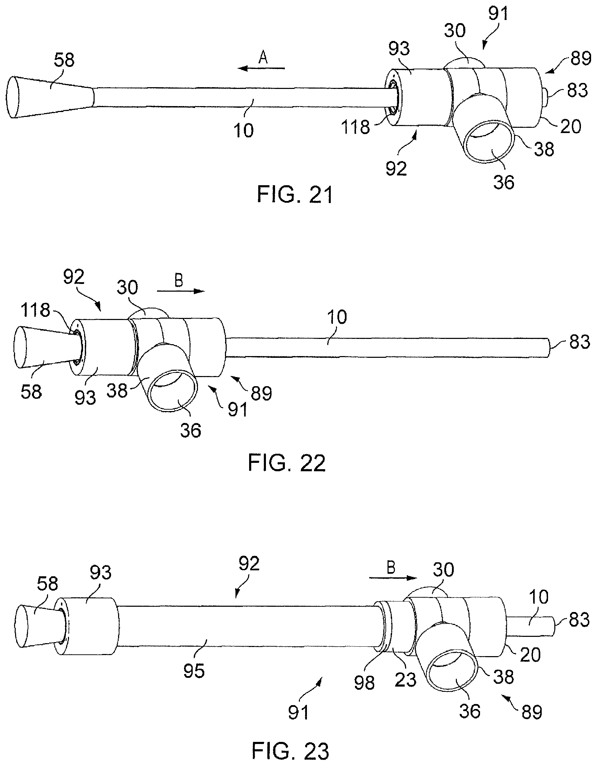

In use, with the adapter 89 disengaged from the face mask 90, and with the shielding sleeve 95 in the stored state, and the first end 96 thereof clamped onto the second main port 23 of the adapter 89 by the coupling ring 98, and the sleeve storing housing 93 engaging the second main port 23 of the adapter 89, the distal end 83 of the instrument or endoscope 10 is entered into the sleeve storing housing 93 through the duckbill valve 118, and in turn through the secondary instrument accommodating bore 110, and then through the instrument accommodating bore 25 of the adapter 89, see Fig. 21. The adapter 89 and the sleeve storing housing 93 still engaged with each other are urged proximally in the direction of the arrow A along the endoscope 10 until the duckbill valve 118 tightly engages the endoscope 10 adjacent the diverging proximal end 58 thereof, see Fig. 22.

Once the duckbill valve 118 is tightly and sealably engaged with the diverging proximal end 58 of the endoscope 10, the adapter 89 is disengaged from the sleeve storing housing 93. The adapter 89 is then urged distally along the endoscope 10 in the direction of the arrow B, see Figs. 22 and 23. As the adapter 89 is being urged distally along the endoscope 10, since the first end 96 of the shielding sleeve 95 is clamped onto the second main port 23 by the coupling ring 98, the shielding sleeve is drawn from the stored state in the storage chamber 94 through the annular access opening 117, defined between the closure member 114 and the end 125 of the secondary tubular member 109, to the extended state extending along and shielding the endoscope 10. The adapter 89 is then engaged with the face mask 90 by engaging the first main port 20 of the adapter 89 with the instrument port 7 of the face mask 90. The face mask 90, if it has not already been secured to the face of the subject, is then secured to the face of the subject, and the endoscope 10 or other instrument is urged through the instrument port 7 of the face mask and is urged orally or nasally into the subject by a surgeon gripping the endoscope through the shielding sleeve 95.

Thereafter, operation and use of the face mask 90 is similar to that already described with reference to the face mask 50 described with reference to Figs. 11 to 18.

Referring now to Figs. 29 to 32 there is illustrated another face mask indicated generally by the reference numeral 130 for accommodating an instrument, for example, an endoscope orally or nasally into a subject for carrying out a procedure on the subject internally in a cavity, lumen or vessel of the subject. The face mask 130 comprises breathing apparatus indicated generally by the reference numeral 132 to allow the subject to breathe through the face mask 130, and to minimise, and preferably, prevent transmission of pathogens, bacteria, infections, viruses and the like in air and gases exhaled or otherwise expelled from the subject entering the environment.

The face mask 130 is substantially similar to the face mask 45 described with reference to Figs. 8 to 10, and similar components are identified by the same reference numerals. The face mask 130 comprises an instrument port 133 for accommodating an instrument, such as an endoscope 10 into and through the face mask 130, and in turn orally or nasally into the subject, and a secondary port 134. The secondary port 134 may be provided for entering another instrument, or a tube for delivering insufflating gas to the cavity, lumen or vessel in which the procedure is being carried out, or to accommodate air for breathing by the subject. The breathing apparatus 132 is engaged in the secondary port 134, leaving the instrument port 133 available to accommodate an instrument, such as the endoscope 10.

The breathing apparatus 132 comprises a housing 135 having a main tubular member 137 terminating at a first end in a main port 138 for releasably and sealably engaging the secondary port 134 of the face mask 130. The main tubular member 137 extends from the main port 138 and terminates at its opposite second end in a first tubular member 139 and a second tubular member 140, both of which extend transversely from the main tubular member 137 on respective opposite sides thereof. The first tubular member 139 terminates in a first air accommodating port, namely, a first secondary port 142, while the second tubular member 140 terminates in a second air accommodating port, namely, a second secondary port 144. A main bore 145 extends through the main tubular member 137 from the main port 138 and terminates in and communicates with first and second bores 146 and 147, respectively, extending through the respective first and second tubular members 139 and 140. The first bore 146 terminates in the first secondary port 142 and communicates the first secondary port 142 with the main port 138 through the main bore 145. The second bore 147 terminates in the second secondary port 144 and communicates the second secondary port 144 with the main port 138 through the main bore 145.

A first non-return valve 149 is coupled to the first secondary port 142 and communicates with the first bore 146. The first non-return valve 149 is adapted to permit fluid flow through the first bore 146 in an outward direction from the main bore 145 through the first secondary port 142 and in turn through the first non-return valve 149 for accommodating air and other gases exhaled or otherwise expelled orally or nasally by the subject. A second non-return valve 150 is coupled to the second secondary port 144 and communicates with the second bore 147. The second non-return valve 150 is adapted to permit fluid flow through the second bore 147 in an inward direction from the second secondary port 144 to the main bore 145 for accommodating air and other gases therethrough for inhaling by the subject.

A first filter 152 which is similar to the first filter 32 of the adapter 14 of the face mask 1 described with reference to Figs. 1 to 6 is coupled to the first non-return valve 149 and communicates with the first bore 146 through the first non-return valve 149 for filtering out particulate matter, including pathogens, bacteria, infections and viruses from air and other gases exhaled or otherwise expelled orally or nasally by the subject, so that the exhaled and expelled air and gases are safely dispersed through a discharge port 153 of the first filter 152.

A second filter 155 is coupled to the second non-return valve 150 and communicates with the second bore 147 through the second non-return valve 150 for filtering pathogens, bacteria, infections and viruses from air for inhaling by the subject. An inlet port 156 of the second filter 155 accommodates air therethrough into the second filter 155. The second filter 155 is similar to the second filter 42 of the adapter 41 of the face mask 40 described with reference to Fig. 7.

In use, with the breathing apparatus 132 engaged in the secondary port 134 of the face mask 130, the face mask 130 is secured to the face of a subject. With the face mask 130 secured to the face of the subject, the subject breathes through the breathing apparatus 132, with air for inhaling being drawn through the second filter 155, the second non-return valve 150, the second bore 147 and the main bore 145. Air and other gases exhaled or otherwise expelled from the subject, is delivered into the environment through the main bore 145, the first bore 146, the first non-return valve 149 and the first filter 152, from which the exhaled air and other gases are dispersed through the discharge port 153 safely into the environment. A surgeon may insert an instrument, for example, the endoscope 10 through the instrument port 133 of the face mask 130 which is then orally or nasally entered into the subject. A suitable sealing means (not shown) is located in the instrument port 133 for slideably and sealably engaging the instrument or the endoscope 10, in order to prevent air and other gases exhaled or otherwise expelled orally or nasally from the subject leaking through the instrument port 133 into the environment.

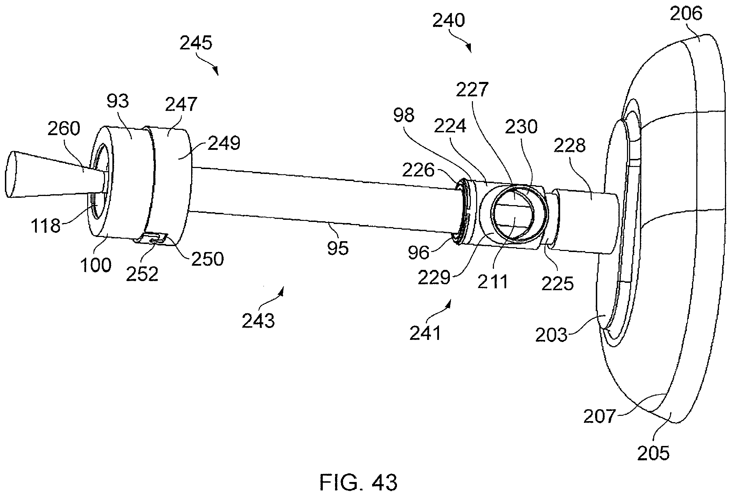

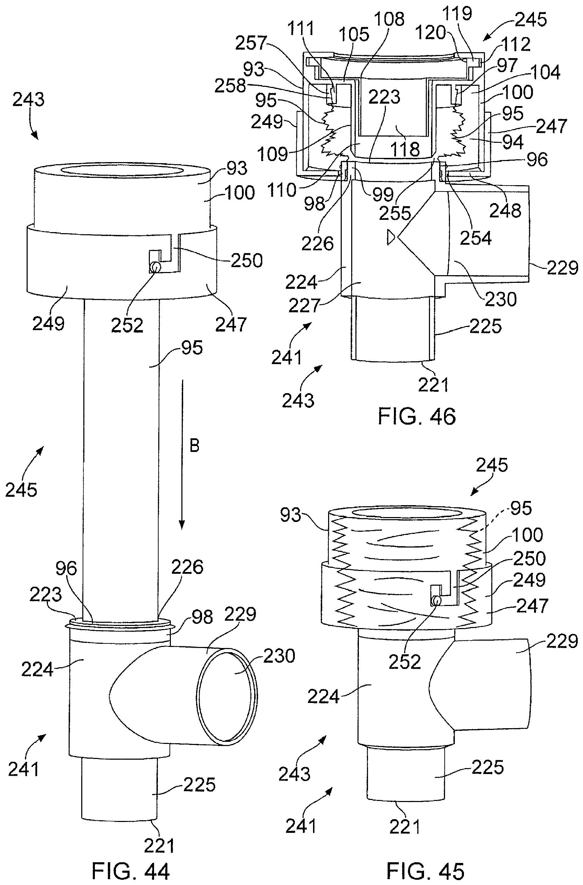

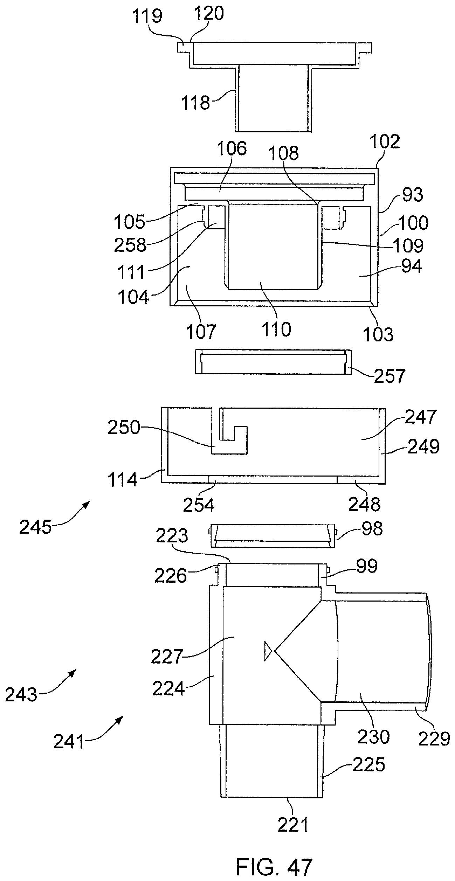

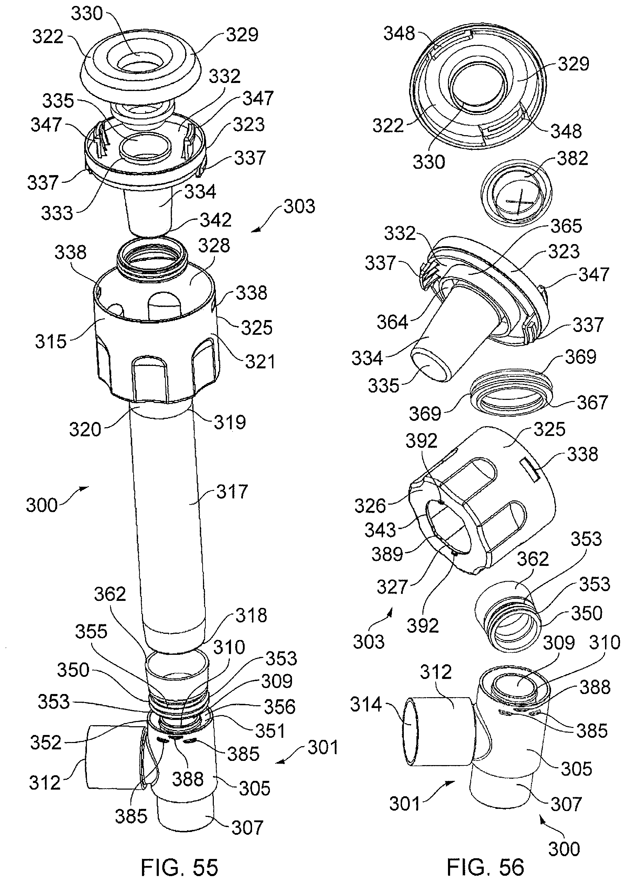

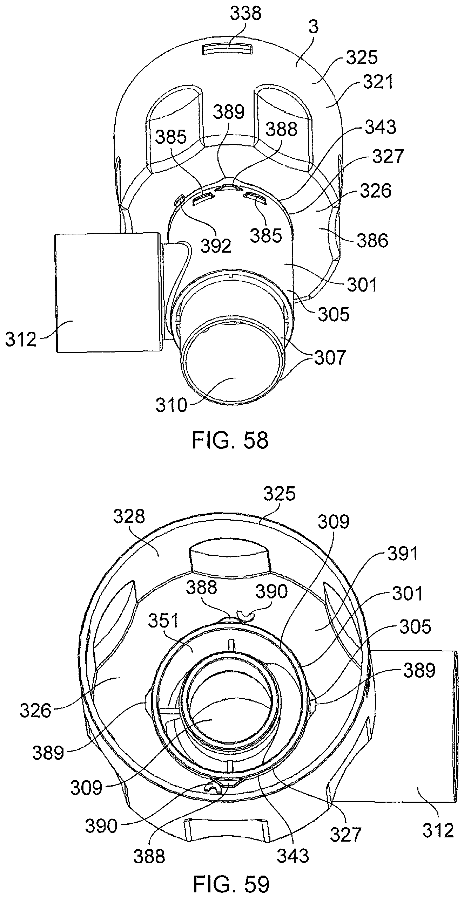

It is envisaged that any of the instrument shielding apparatus described with reference to Figs. 11 to 18 and Figs. 19 to 28, and the instrument shielding apparatus which will be described with reference to Figs. 43 to 66 may be engaged directly in the instrument port 133 of the face mask 130 for shielding the instrument, for example, the endoscope 10 being entered through the instrument port 133.

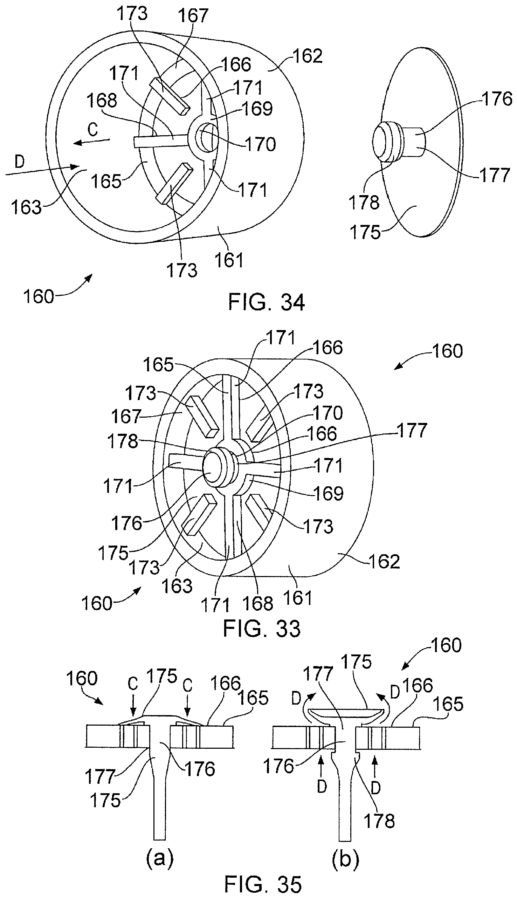

Referring now Figs. 33 to 35 there is illustrated a non-return valve indicated generally by the reference numeral 160, which may be used as a non-return valve for any of the first and second non-return valves of the adapters and the breathing apparatus described herein. Although, needless to say, it will be understood by those skilled in the art that other types of non-return valves may be used in adapters and the breathing apparatus.

The non-return valve 160 comprises an umbrella valve comprising a hollow cylindrical housing 161 formed by a cylindrical side wall 162, and defining a bore 163 extending therethrough. A perforated support 165 extends transversely in the bore 163 and defines a valve seat 166. The perforated support 165 comprises an outer ring member 167 extending circumferentially around the side wall 162 and radially inwardly into the bore 163. A cruciform member 168 having an inner central ring member 169 defining a central bore 170 extending therethrough, and four radial members 171 extending at substantially 90° to each other from the inner central ring member 169 extend to the outer ring member 167. Four inwardly extending radial members 173 extend inwardly from the outer ring member 167 towards the central ring member 169 but stop short of the inner central ring member 169. The inwardly extending radial members 173 define an angle of 45° with their adjacent radial members 171.

A flexible disc valve member 175 is anchored in the inner central ring member 169 by an anchor member 176 and abuts the valve seat 166 defined by the perforated support member 165 to prevent fluid flow through the perforated support 165 in the direction of the arrow C from the side of the perforated support 165 adjacent the flexible disc valve member 175 to the other side of the perforated support 165. Additionally, the flexible disc valve member 175 is deformable to disengage the valve seat 166 to permit fluid flow through the perforated support 165 in the reverse direction, namely, in the direction of the arrow D. The anchor member 176 comprises a spigot 177 extending centrally from the flexible disc valve member 175 for engaging the central bore 170 extending through the inner central ring member 169 for locating the flexible disc valve member 175 centrally in the bore 163 extending through the housing 161. The spigot 177 terminates in a deformable resilient head 178 which retains the spigot 177 in the central bore 170 of the central ring member 169 with a snap-fit action.

The flexible disc valve member 175 is sufficiently flexible to flex away from the valve seat 166 defined by the perforated support 165 in response to air being exhaled or inhaled as the case may be and passing through the perforated support 165 in the direction of the arrow D in order to permit fluid flow through the perforated support 165 and in turn through the non-return valve 160 in the direction of the arrow D. However, the flexibility of the disc valve member 175 is such as to be responsive to air being exhaled or inhaled as the case may be flowing in the direction of the arrow C so that the flexible disc valve member 175 is urged against the valve seat 166 defined by the perforated support 165 in order to prevent flow of air through the perforated support 165 and in turn through the non-return valve 160 in the direction of the arrow C. The operating principle of the non-return valve 160 is illustrated in the diagrammatic views of the non-return valve 160 of Figs. 35(a) and 35(b). In Fig. 35(a) the non-return valve 160 is illustrated with the disc valve member 175 abutting the perforated support 165, thereby preventing fluid flow therethrough in the direction of the arrows C, and in Fig. 35(b), the disc valve member 175 is illustrated deformed and spaced apart from the valve seat 166 defined by the perforated support 165, thereby permitting fluid flow through the non-return valve 160 in the direction of the arrows D.

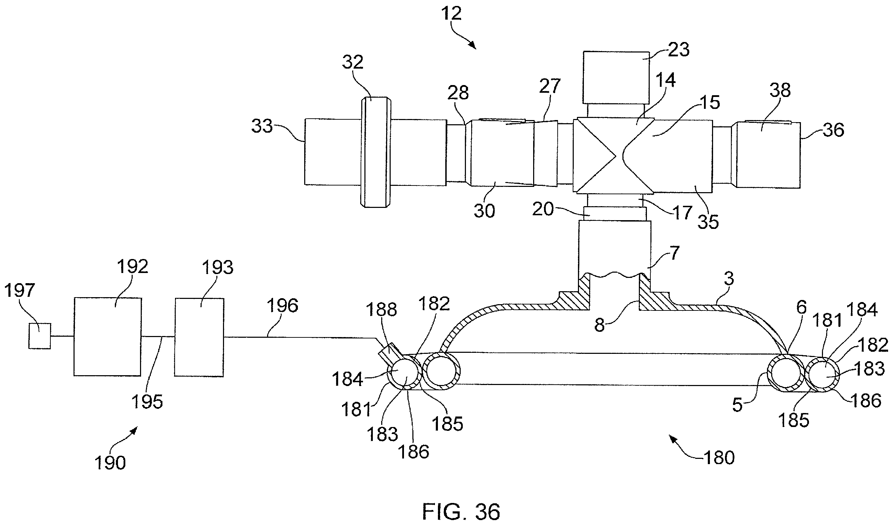

Referring now to Fig. 36, there is illustrated another face mask indicated generally by the reference numeral 180 for accommodating an instrument, for example, the endoscope 10 therethrough orally or nasally into a vessel or cavity of a subject. The face mask 180 is substantially similar to the face mask 1, and similar components are identified by the same reference numerals. The main difference between the face mask 180 and the face mask 1 is that a collector 181 is secured to and extends around the outer peripheral tubular portion 5 of the face mask 180 for collecting any air and other gases exhaled or otherwise expelled orally and nasally from the subject which may leak around the outer peripheral tubular portion 5 of the face mask 180 between the face mask 1 and the face of the subject.

The collector 181 comprises an endless collecting conduit 182 extending around and secured to the outer peripheral tubular portion 5 of the face mask 180. The collecting conduit 182 defines an endless bore 183 extending therethrough which defines a communicating chamber 184. A plurality of inlet ports 185 spaced apart longitudinally along the collecting conduit 182 extend radially from the communicating chamber 184 and terminate in the external surface 186 of the collecting conduit 182, and are arranged in the collecting conduit 182 adjacent the outer peripheral tubular portion 5 of the face mask 1, in order to collect any escaping air and gases which may leak between the outer peripheral tubular portion 5 of the face mask 180 and the face of the subject.

An outlet port 188 extending from the collecting conduit 182 and communicating with the inlet ports 185 through the communicating chamber 184 is adapted for coupling to a vacuum system 190 for in turn applying a vacuum to the inlet ports 185 for drawing any air and gases leaking from the face mask 1 around the outer peripheral tubular portion 5 thereof between the outer peripheral tubular portion 5 and the face of the subject.

The vacuum system 190 comprises a vacuum pump 192 and a third filter 193. The vacuum pump 192 is connected to the third filter 193 by a vacuum line 195. A vacuum line 196 connects the third filter 193 to the outlet port 188 from the collecting conduit 182 for applying a vacuum to the inlet ports 185. Air and other gases leaking from the face mask 180 around the outer peripheral tubular portion 5 thereof, is drawn under vacuum through the inlet ports 185 for filtering thereof by the third filter 193. The third filter 193 may be any suitable filter for filtering out pathogens, bacteria, infections and viruses, and in this case is similar to the first filter 32 for filtering out the pathogens, bacteria, viruses and infections from the leaked air and gases. The filtered air is then safely delivered into the environment through an exhaust port 197 of the vacuum system 190.

The vacuum pump 192 is configured to generate a vacuum of pressure in the range of 150mmHg to 300mmHg below atmospheric pressure, so that an adequate relatively light vacuum is drawn on the inlet ports 185 for drawing air and other gases leaking from the face mask between the face mask and the face of the subject into the endless bore 183 of the endless connecting conduit 182.

In use, the face mask 180, with the adapter 14 sealably engaged in the main port 7, is secured to the face of a subject as already described. The outlet port 188 from the collecting conduit 182 is connected by the vacuum line 196 to the third filter 193 and through the vacuum line 195 to the vacuum pump 192. On activating the vacuum pump 192, a vacuum is applied to the inlet ports 185, and any air and other gases leaking from the face mask 180 between the outer peripheral tubular portion 5 and the face of the subject are drawn by the vacuum through the inlet ports 185 and in turn through the filter 193 where pathogens, bacteria, viruses and infections are filtered out from the air and other gases, and the air and other gases are then dispersed safely into the environment through the exhaust port 197 from the vacuum pump 192. The subject breathes through the adapter 14 as already described with reference to the face mask 1, and the endoscope or other instruments are passed through the instrument bore 25, and in turn the passageway 8 through the main port 7 of the face mask 180 for oral or nasal entry into the subject.

Otherwise, the face mask 180 and its use is similar to that of the face mask 1.

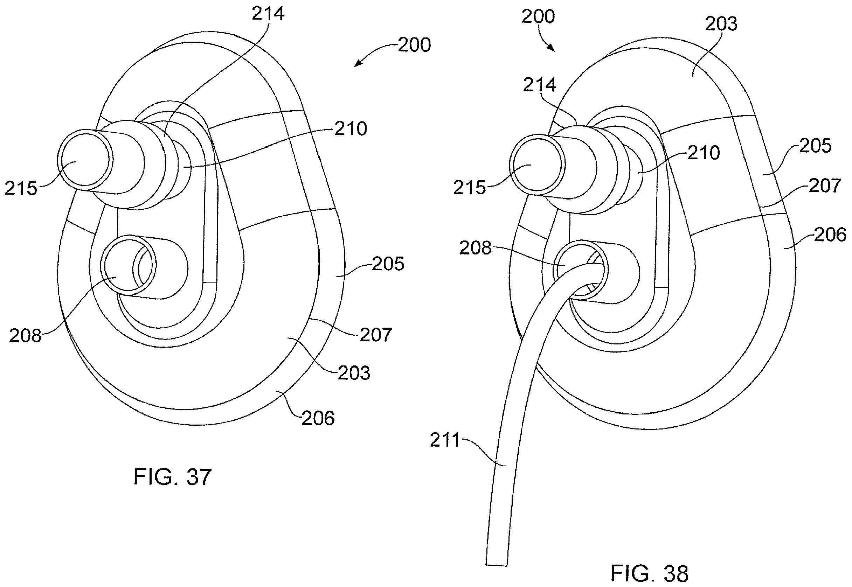

Referring now to Figs. 37 and 38, there is illustrated a face mask indicated generally by the reference numeral 200, for securing to a face of a subject during the carrying out of a procedure, for example, an endoscopic or other procedure requiring an endoscope or other instrument to be entered orally or nasally into a subject. The face mask 200 comprises a central portion 203 and a cushion portion 205 comprising an endless cushion tube 206 extending around an outer periphery 207 of the central portion 203 thereof. The cushion tube 206 is air filled in order to provide a
soft cushion to form a seal around the periphery of the face mask between the mask and the face of a subject. As described with reference to the face masks of Figs. 1 to 36, the cushion tube 206 may be prefilled with air or gas, or may be provided for pumping-up with air or gas through a suitable valve, as will be well-understood by those skilled in the art. The face mask 200 like the face masks described with reference to Figs. 1 to 36 is sized and shaped to cover the mouth and nose of a subject, and to extend around the chin of the subject and upwardly along the cheeks of the subject and across the bridge of the nose. The face mask 200 like the face masks described with reference to Figs. 1 to 36 may be secured to the face of a subject in any suitable manner, for example, by side loops extending around the ears of a subject, or by straps extending around and behind the head of a subject.

Two ports, namely, an instrument port 208, and an air accommodating port 210 are formed in the central portion 203 of the face mask 200 and are spaced apart from each other in the central portion 203. The instrument port 208 is configured to accommodate an instrument, for example, an endoscope 211 through the face mask for oral or nasal entry into the subject. The air accommodating port 210 is provided to accommodate air and other gases to the subject for inhaling, and for accommodating air exhaled by the subject, and any other gases which may be exhaled by the subject or expelled orally or nasally from the subject.

A filter 214 is releasably and sealably engaged in the air accommodating port 210 for filtering particulate matter, and in particular, pathogens, bacteria, infections and viruses entrained in air and other gases passing through the air accommodating port 210, either passing inwardly through the air accommodating port 210 for inhaling by the subject, or passing outwardly through the air accommodating port 210 being exhaled or expelled from the subject. Filtered air and gases are safely dispersed from the filter 214 into the atmosphere through an outlet port 215 from the filter 214. The filter 214 is similar to the filters described with reference to the face masks of Figs. 1 to 36, and comprises a blended synthetic fibre material housed in a polypropylene housing, and is adapted to filter out particulate matter of size greater than 0.05 microns entrained in the air or gas passing therethrough.

In cases where the face mask 200 is configured to be disposable, it is envisaged that instead of being releasably engageable in the air accommodating port 210, the filter 214 may be permanently sealably secured in the air accommodating port 210. Additionally, in embodiments of the face mask 200 where the face mask is provided to be disposable, it is envisaged that a filter housing may be integrally formed with the central portion 203 of the face mask, and a filter would be inserted subsequently in the filter housing.

Turning now to the instrument port 208, as discussed above the instrument port 208 is configured for accommodating an instrument, for example, the endoscope 211 therethrough for oral or nasal entry into the subject. Although not illustrated, a sealing means is provided in the instrument port 208 for sealably and slideably engaging the instrument or endoscope 211 as it is being manoeuvred in and urged through the instrument port 208 into the subject or removed therefrom. Any suitable sealing means may be provided in the instrument port 208, for example, a multi-leaf valve, a duckbill valve or any other suitable sealing means.

It is also envisaged in some embodiments of the invention that any of the instrument shielding apparatus according to the invention, or any of the instrument shielding devices according to the invention described herein, may be coupled to the instrument port 208 and to an instrument, for example, the endoscope 211 for shielding the instrument or endoscope 211, and also for providing an airtight seal between the instrument port 208 and the instrument or endoscope 211.

In use, with the face mask secured to the face of the subject, the subject inhales air through the air accommodating port 210 and the filter 214, and exhaled air and other orally or nasally expelled gases exit through the air accommodating port 210 and in turn the filter 214. An instrument or the endoscope 211 is inserted orally or nasally into the subject through the instrument port 208.

Accordingly, air exhaled by the subject and other gases expelled orally or nasally from the subject are filtered through the filter 214, and particulate matter of size greater than 0.05 microns, including pathogens, bacteria, infections and viruses, which may be entrained in the exhaled air or expelled gases from the subject, are filtered out of such exhaled air or expelled gases in the filter 214, so that the exhaled air and expelled gases are dispersed safely into the atmosphere through the outlet port 215 from the filter 214. Additionally, particulate matter of size greater than 0.05 microns including pathogens, bacteria, infections and viruses are filtered out of air being drawn through the filter 214 for inhaling by the subject.

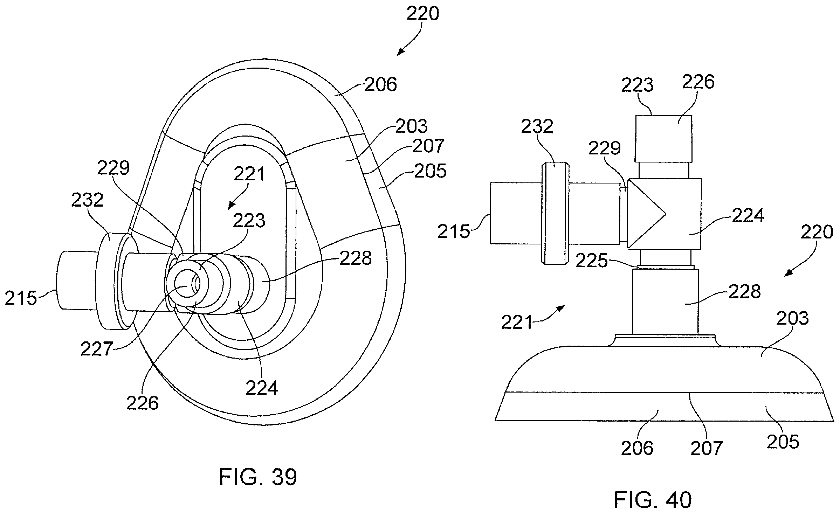

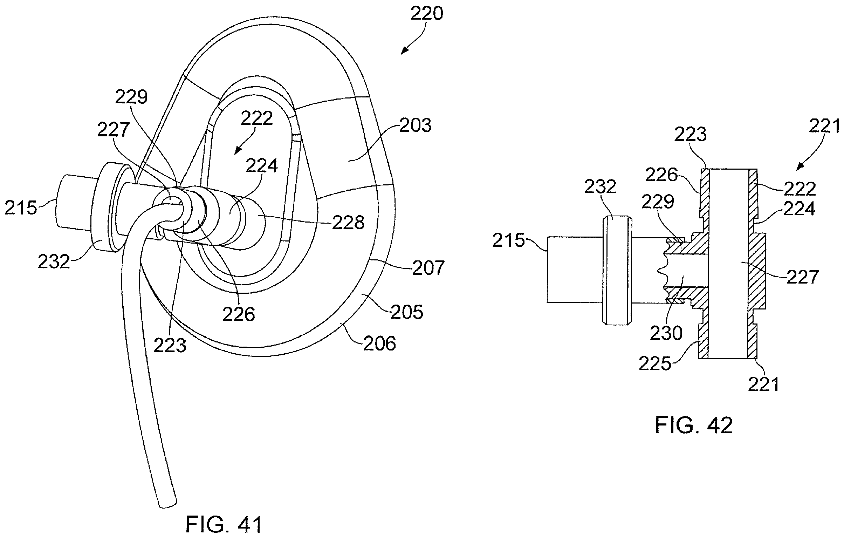

Referring now to Figs. 39 to 42, there is illustrated a face mask indicated generally by the reference numeral 220. The face mask 220 is somewhat similar to the face mask 200, and similar components are identified by the same reference numerals. However, the face mask only comprises an instrument port 228 located in the central portion 203 of the face mask 220. An air accommodating port
is omitted from the central portion 203 of the face mask 220.

An adapter indicated generally by the reference numeral 221, for accommodating an instrument, for example, the endoscope 211 therethrough to the face mask 220, comprises an instrument accommodating housing 222. The instrument accommodating housing 222 comprises a main tubular member 224 extending between a first end 219 and a second end 223. The first end 219 of the main tubular member 224 terminates in a first main port 225 for engaging the instrument port 228 of the face mask 220. The second end 223 of the main tubular member 224 terminates in a second main port 226 for accommodating an instrument, for example, the endoscope 211 into the main tubular member 224. An instrument accommodating bore 227 extends through the main tubular member 224 and communicates the second main port 226 with the first main port 225 for accommodating an instrument, for example, the endoscope 211 from the second main port 226 to the first main port 225, and in turn through the face mask 220 for oral or nasal entry into the subject.

An air accommodating port 229 is teed-off from the main tubular member 224 intermediate the first main port 225 and the second main port 226. A first bore, namely, an air accommodating bore 230 communicates the air accommodating port 229 with the instrument accommodating bore 227 for communicating the air accommodating port 229 with the first main port 225, for in turn accommodating air through the air accommodating port 229 to the subject for inhaling thereof, and for accommodating air and other gases exhaled or expelled from the subject. The diameter of the instrument accommodating bore 227 extending through the main tubular member 224 is sufficient to accommodate air through the instrument accommodating bore 227 along with the instrument or endoscope 211 between the air accommodating port 229 and the first main port 225. The first main port 225 of the instrument accommodating housing 222 is configured to releasably and sealably engage the instrument port 228 of the face mask 220, although it is envisaged that the first main port 225 may be permanently and sealably engaged in the instrument port 228. In other embodiments it is envisaged that the instrument accommodating housing 222 may be integrally formed with the central portion 203 of the face mask 220.

A filter 232 similar to the filter 214 of the face mask 200 is releasably and sealably engaged in the air accommodating port 229 for filtering air being inhaled by the subject and air being exhaled and other gases expelled orally or nasally from the subject. However, it is envisaged that in some embodiments the filter 232 may be permanently sealably secured to the air accommodating port 229. In
other embodiments, it is envisaged that a filter housing may be integrally formed with the instrument accommodating housing 222, and may also be integrally formed with the instrument accommodating housing 222 and the central portion 203 of the face mask 220, and in which case, provision would be made for placing the filter in the filter housing.

A sealing means (not shown) is located in the second main port 226 of the housing 222 for sealably and slideably engaging the instrument or endoscope 211 as it is being manoeuvred and urged inwardly and outwardly therethrough. Additionally, or alternatively, as described with reference to the face mask 200, any one of the instrument shielding devices described herein may be sealably secured to the second main port 226 of the adapter 221 and to the instrument, for example, the endoscope 211 as already described with reference to the embodiments of Figs. 11 to 28.

Use of the face mask 220 is similar to use of the face mask 200. When the face mask 220 is sealably secured to the face of a subject, the subject breathes through the air accommodating port 229 and the filter 232, and exhaled air and gases expelled from the subject exit through the air accommodating port 229 and in turn through the filter 232, where the filtered air and gases are dispersed safely into the environment.

The instrument or endoscope 211 is inserted through the second main port 226 of the instrument accommodating housing 222, and in turn through the instrument accommodating bore 227 of the instrument accommodating housing 222 and then orally or nasally into the subject.

Referring now to Figs. 43 to 50, there is illustrated a face mask according to another embodiment of the disclosure indicated generally by the reference numeral 240. The face mask 240 comprises an instrument shielding apparatus according to the invention indicated generally by the reference numeral 243. The instrument shielding apparatus 243 comprises an adapter also according to the invention and indicated by the reference numeral 241 for accommodating an instrument, for example, an endoscope 211 to and through the face mask 240, and an instrument shielding device which is also according to the invention and indicated generally by the reference numeral 245 for shielding the instrument, for example, the endoscope 211. The instrument shielding device is substantially similar to the instrument shielding device 92 described with reference to Figs. 19 to 28, and similar components are identified by the same reference numerals. The adapter 241 comprises an instrument accommodating housing 242 through which the instrument or endoscope 211 is urged to the face mask 240. The instrument accommodating housing 242 of the adapter 241 is substantially similar to the instrument accommodating housing 222 of the adapter 221 described with reference to Figs. 39 to 42, and components of the instrument accommodating housing 242 which are similar to those of the instrument accommodating housing 222 are identified by the same reference numerals. The face mask 240 is substantially similar to the face mask 220 described with reference to Figs. 39 to 42, and similar components are identified by the same reference numerals.

For convenience, the filter 232 has been omitted from the air accommodating port 229 of the adapter 241, however, it is to be understood that a filter similar to the filter 232 of the adapter 221 will be sealably engaged in the air accommodating port 229 of the adapter 241. The instrument accommodating housing 242 of the adapter 241 is releasably and sealably engaged with the face mask 240 by sealably engaging the first main port 225 of the instrument accommodating housing 242 with the instrument port 228 of the face mask 240. A suitable sealing means, which is not shown in the drawings, is located in the second main port 226 or in the instrument accommodating bore 227 of the adapter 241 between the first bore, namely, the air accommodating bore 230 and the second main port 226 for sealably and slideably engaging the instrument or endoscope in the second main port 226 or in the instrument bore 227 in order to prevent passage of air between the instrument or endoscope 211 and the second main port 226.

Turning now to the instrument shielding device 245, the only differences between the instrument shielding device 245 and the instrument shielding device 92 of Figs. 19 to 28 lies in the sleeve storing housing 93. in this embodiment of the invention the closure member 114 is releasably secured to the sleeve storing housing 93, in order to allow the shielding sleeve 95 to be returned into the storage chamber 94 and to be stored therein after use. In this case the closure member 114 is provided in the form of a bayonet cap 247.

The bayonet cap 247 comprises an end plate 248 and a cylindrical peripheral wall 249 extending around and from the end plate 248. A pair of bayonet slots 250 are formed in the peripheral wall 249 spaced apart circumferentially around the peripheral wall 249 at 180° intervals for engaging corresponding bayonet pins 252 extending transversely from the cylindrical outer side wall 100 of the sleeve storing housing 93, in order to releasably secure the bayonet end cap 247 to the sleeve storing housing 93 for closing the storage chamber 94. A central access opening 254, similar to the central access opening 115 of the closure member 114 of the instrument shielding device 92, is formed in the end plate 248 for defining with the end 125 of the secondary tubular member 109 an annular opening 255 for accommodating the shielding sleeve 95 from the storage chamber 94. The diameter of the central access opening 254 is such that the end plate 248 slideably engages the coupling ring 98 on the second main port 226 of the instrument accommodating housing 242, so that the sleeve storing housing 93 is releasably engageable with the instrument accommodating housing 242 when the shielding sleeve 95 is in the stored state in the storage chamber 94.

A clamping ring 257, similar to the clamping ring 112 of the instrument shielding device 92, extends around an outer surface 258 of the intermediate cylindrical wall 111 and sealably clamps the second end 97 of the shielding sleeve 95 securely between the intermediate cylindrical wall 111 and itself, for in turn securing the second end 97 of the shielding sleeve 95 to the sleeve storing housing 93 in the storage chamber 94.

In use, it is envisaged that the instrument shielding apparatus 243 comprising the adapter 241 and the instrument shielding device 245 will be supplied as a single unit with the instrument shielding device 245 releasably engaged with the adapter 241 with the bayonet cap 247 releasably engaged with the second main port 226, and with the first end 96 of the shielding sleeve 95 sealably clamped onto the second main port 226 by the coupling ring 98. Typically, the instrument shielding apparatus 243 will be supplied separately of the face mask 240. Although, in some embodiments of the invention it is envisaged that the instrument shielding apparatus 243 may be sold with the face mask 240 as a single unit with the first main port 225 of the adapter 241 releasably engaged in the instrument port 226 of the face mask 240.

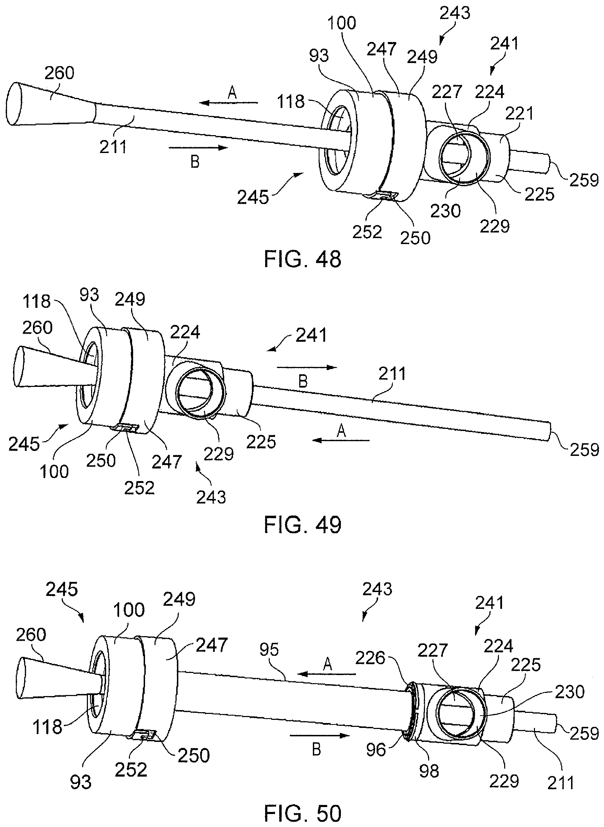

Accordingly, in use with the adapter 241 disengaged from the face mask 240, and with the instrument shielding device 245 engaged with the second main port 226 of the adapter 241, and with the first end 96 of the shielding sleeve 95 secured to the second main port 226 of the instrument accommodating housing 242 by the coupling ring 98, the distal end 259 of the endoscope 211 is entered into the duckbill valve 118 in the secondary instrument accommodating bore 110 of the sleeve storing housing 93, and in turn into the instrument accommodating bore 227 of the adapter 241. The instrument shielding device 245 and the adapter 241, engaged with each other, are urged proximally along the endoscope 211 in the direction of the arrow A as illustrated in Figs. 48 and 49, until the duckbill 118 tightly engages the proximal diverging end 260 of the endoscope 211. The adapter 241 is disengaged from the bayonet cap 247 of the instrument shielding device 245 and is urged distally in the direction of the arrow B along the endoscope 211 towards the distal end 259 thereof. As the adapter 241 is being urged distally along the endoscope 211, the shielding sleeve 95 is drawn from the storage chamber 94 through the annular opening 255 defined between the bayonet cap 247 and the end 125 of the secondary tubular member 109 of the sleeve storing housing 93. Once the adapter 241 has been urged to the distal end 259 of the endoscope 211 with the distal end 259 thereof extending through the first main port 225, the first main port 225 is engaged in the instrument port 228 of the face mask 240. The face mask 240 is then secured to the face of the subject as already described with reference to the face mask 220.

The subject breathes through the air accommodating port 229 and the filter 232, which although not illustrated, as discussed above, is located sealably engaged in the air accommodating port 229 of the adapter 241. The endoscope 211 is then urged orally or nasally into the subject by gripping the instrument or endoscope 211 through the shielding sleeve 95 as already described with reference to the instrument shielding device 92 described with reference to Figs. 19 to 28.

As discussed above a suitable sealing means is located in the second main port 226 or in the instrument accommodating bore 227 of the adapter 241 for sealably and slideably engaging the instrument or the endoscope 211. The provision of this sealing means prevents the passage of air being exhaled or expelled orally or nasally by the subject past the second main port 226, which would otherwise result in the shielding sleeve 95 being inflated around the endoscope 211 in the instrument accommodating lumen 101. Such a sealing means may comprise a duckbill valve, a multi-leaf valve or any other sealing means suitable for sealably and slideably engaging the instrument or endoscope 211 in the second main port 226 or in the instrument accommodating bore 227 between the air accommodating bore 230 and the second main port 226 of the adapter 241.

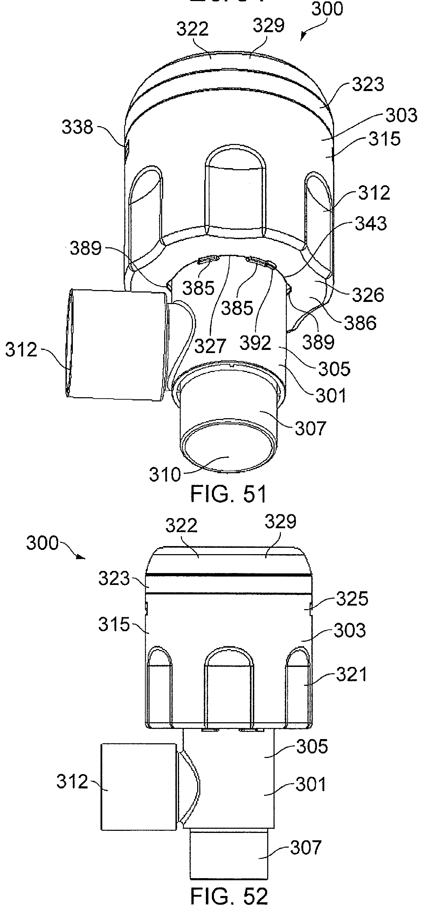

Referring now to Figs. 51 to 63 there is illustrated an instrument shielding apparatus also according to the invention and indicated generally by the reference numeral 300 for coupling to a face mask, for example, a face mask similar to the face mask 1 described with reference to Figs. 1 to 6 thereof. The instrument shielding apparatus 300 comprises an adapter also according to the invention and indicated generally by the reference numeral 301 for coupling to an instrument port 7 of the face mask 1. The instrument shielding apparatus 300 also comprises an instrument shielding device also according to the invention and indicated generally by the reference numeral 303 secured to the adapter 301 for accommodating and shielding an instrument, for example, an endoscope, such as the endoscope 10 described with reference to Figs. 1 to 6 for entry through an instrument port of the face mask.

The adapter 301 comprises a tubular instrument accommodating housing 305 terminating at one end in a first main port 307 for engaging the instrument port of the face mask, and at the other end, in a second main port 309. An instrument accommodating bore 310 extending through the instrument accommodating housing 305 extends from and communicates with the first main port 307 and the second main port 309. The instrument accommodating bore 310 is sized for accommodating an instrument, for example, an endoscope therethrough and in turn through the first main port 307 to the face mask for oral or nasal entry into a subject. An air accommodating port 312 is T'd off from the instrument accommodating housing 305 and communicates with the instrument accommodating bore 310 through a first bore 314 for accommodating air and other gases through the first bore 314, the instrument bore 310 and the first main port 307 for in turn accommodating air and other gases to and from a subject to which the face mask is secured. A filter (not shown), similar to the first filter 32 described with reference to the face mask 1 of Figs. 1 to 6 is sealably engaged in the air accommodating port 312 for filtering out pathogens, bacteria, infections and viruses from air and gases passing through the filter (not shown).

The instrument shielding device 303 comprises a sleeve storing housing 315 for storing a shielding sleeve 317 therein for shielding the endoscope or other instrument extending therethrough. The shielding sleeve 317 is similar to the shielding sleeves 55 and 95 of the instrument shielding devices 54 and 92 described with reference to Figs. 11 to 28. Like the shielding sleeves 55 and 95, the shielding sleeve 317 extends between a first end 318 and a second end 319 and defines an instrument accommodating lumen 320 extending from the first end 318 to the second end 319 thereof for accommodating the instrument therethrough.

In this embodiment of the invention the shield storing housing 315 comprises a three-part housing having a first part 321, a second part 322 and a third part 323. The first part 321 comprises a cylindrical outer side wall 325 extending around and from a base plate 326, which together define a chamber 328. A central access opening 327 is formed in the base plate 326. The second part 323 of the sleeve storing housing 315 comprises an end cap 329 having an instrument receiving bore 330 extending therethrough.

The third part 323 of the sleeve storing housing 315 is located intermediate the first part 321 and the second part 322, and comprises an intermediate plate member 332 having a central opening 333 extending therethrough. An elongated instrument accommodating tubular member 334 extends through the central opening 333 and is formed with the intermediate plate member 332. The instrument accommodating tubular member 334 defines an instrument accommodating bore 335 extending therethrough for accommodating an instrument through the sleeve storing housing 315. A pair of first engagement clips 337 extend from the intermediate plate member 332, and are spaced apart at 180° around the intermediate plate member 332 for engaging corresponding first receiving openings 338 in the outer side wall 325 of the first part 321.

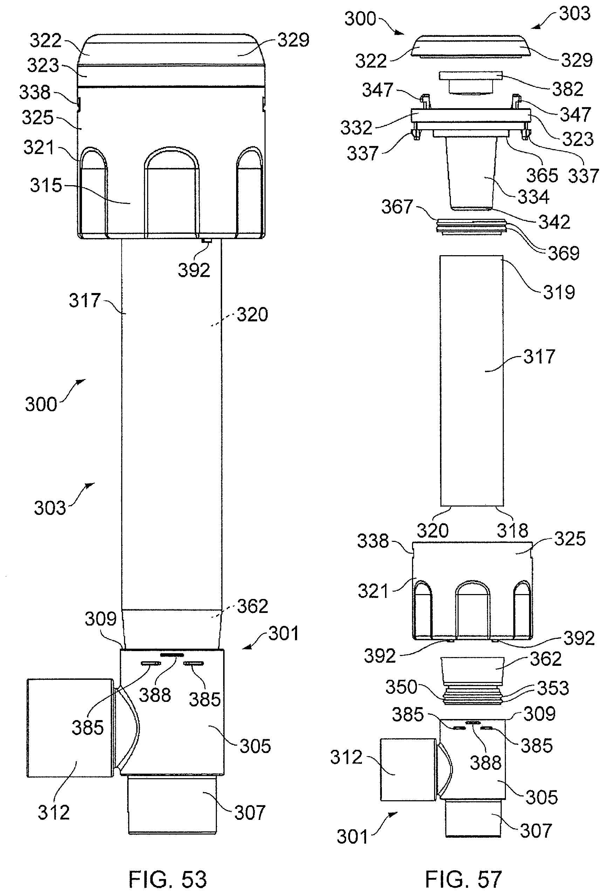

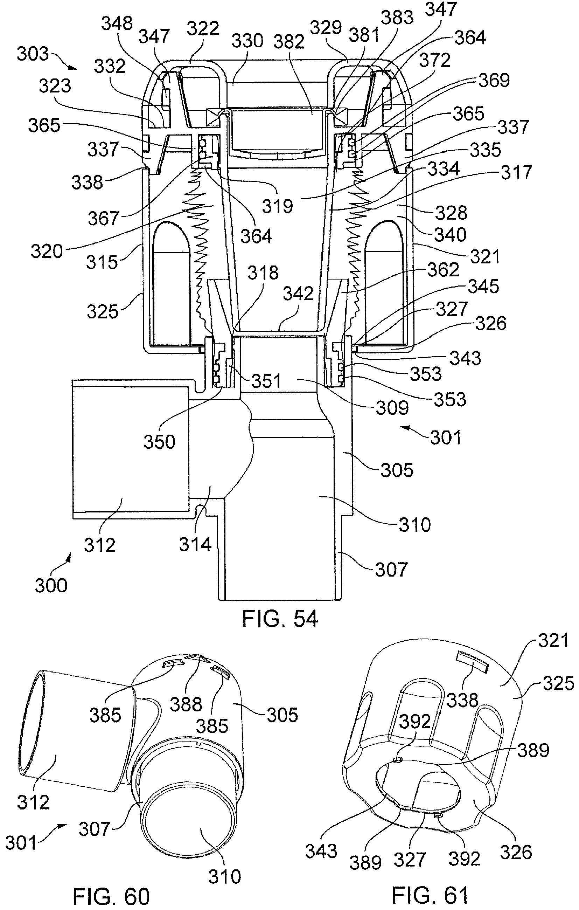

When the intermediate plate member 332 is secured in the first part 321 of the sleeve storing housing 315, the instrument accommodating tubular member 334 extending from the intermediate plate member 332 extends into the chamber 328 of the first part 321, and defines with the outer side wall 325 and the base plate 326, an annular storage chamber 340. The shielding sleeve 317 is stored in the storage chamber 340 in a collapsed stored state, illustrated in Fig. 54, with the instrument accommodating tubular member 334 extending through the instrument accommodating lumen 320 of the shielding sleeve 317. The instrument accommodating tubular member 334 terminates in a free end 342, which defines with a rim 343 of the central access opening 327 of the base plate 326, an annular outlet opening 345 for accommodating the shielding sleeve 317 from the stored state within the storage chamber 340 to an extended state, illustrated in Fig. 53, for shielding the instrument as will be described below.

A pair of second engagement clips 347 spaced apart at 180° intervals around the intermediate plate member 332 extend from the intermediate plate member 332, and engage corresponding latching grooves 348 formed in the end cap 329 for securing the end cap 329 to the intermediate plate member 332.

Turning now to the shielding sleeve 317, the shielding sleeve 317 is sealably secured to the second main port 309 of the adapter 301 by a first coupling element, which in this embodiment of the invention comprises a coupling ring 350 which is tightly and securely engageable in an annular receiving groove 351 extending into the instrument accommodating housing 305 adjacent and around the second main port 309 at an end 352. A pair of spaced apart O-rings 353 located in annular recesses 354 extending around the outer periphery 355 of the coupling ring 350 cooperate with a surface 356 of the annular receiving groove 351 for tightly and sealably clamping a portion 358 of the shielding sleeve 317 adjacent the first end 318 thereof adjacent the surface 356 of the annular receiving groove 351. The coupling ring 350 is configured so that a portion 359 of the shielding sleeve 317 adjacent the first end 318 thereof is returned inwardly into the coupling ring 350, so that the portions 358 and 359 of the shielding sleeve 317 define an annular recess 360 within which the coupling ring 350 is located for tightly and sealably securing the first end 318 of the shielding sleeve 317 in the annular receiving groove 351 of the second main port 309 of the adapter 301. A retaining ring 357 extending into the annular receiving groove 351 is engageable with an engagement ring 361 extending inwardly from the coupling ring 350 for retaining the coupling ring 350 in the annular receiving groove 351. A diverging collar 362 concentric with the coupling ring 350 diverges outwardly from the coupling ring 350 into the instrument accommodating lumen 320 of the shielding sleeve 317 adjacent the first end 318 thereof for spacing the shielding sleeve 317 from the instrument as the instrument enters the second main port 309 of the adapter 301.

The second end 319 of the shielding sleeve 317 is secured to the intermediate plate member 332 in the storage chamber 340 in an annular receiving groove 364 formed in the storage chamber 340 between the instrument accommodating tubular member 334 and an intermediate cylindrical wall 365 extending from the intermediate plate member 332 into the storage chamber 340 spaced apart from the instrument accommodating tubular member 334. A clamping ring 367 is tightly engageable in the annular receiving groove 364 for tightly and sealably clamping the second end 318 of the shielding sleeve 317 in the annular receiving groove 364. A pair of spaced apart O-rings 369 are located in annular recesses 370 extending around the outer periphery of the clamping ring 367 and cooperate with the clamping ring 367 and an inner surface 372 of the intermediate wall 365 for tightly clamping the shielding sleeve 317 adjacent the second end 319 in the annular receiving groove 364. The clamping ring 367 like the coupling ring 350 is adapted to accommodate a portion 374 of the shielding sleeve 317 adjacent the second end 319 thereof to return inwardly into the clamping ring 367, so that the returned portion 374 of the shielding sleeve 317 forms with a portion 375 of the shielding sleeve 317, which is clamped between the O-rings 369 and the intermediate wall 365, an annular recess 377 within which the clamping ring 367 is located. A retaining ring 379 extending around the instrument accommodating tubular member 374 and into the annular receiving groove 364 is engageable with an inwardly extending engagement ring 380 extending inwardly from the clamping ring 367 for securely retaining the clamping ring 367 in the annular receiving groove 364.

A second coupling element for tightly and sealably engaging an instrument, for example, an endoscope in the instrument accommodating bore 335 of the instrument accommodating tubular member 334 comprises a cross-slit valve 382. The cross-slit valve 382 is located in the instrument accommodating bore 337 extending through the instrument accommodating tubular member 334 adjacent the instrument receiving bore 330 formed in the end cap 329. In the case of the instrument being an endoscope, the duckbill valve 382 is adapted for engaging the endoscope adjacent a diverging proximal end thereof, such as the proximal end 58 of the endoscope 10 described in connection with the instrument shielding device 92 described with reference to Figs. 19 to 28. A circumferential flange 381 extending circumferentially around and radially outwardly from the cross-slit valve 382 is engaged between an end 383 of the instrument accommodating tubular member 334 and the end cap 329 for securing the cross-slit valve 382 in the instrument accommodating bore 335 defined by the instrument accommodating tubular member 334.

The sleeve storing housing 315 is releasably secured to the adapter 301 with the central access opening 327 in the base plate 326 of the first part 321 of the sleeve storing housing 315 engaging the instrument accommodating housing of the adapter 301 adjacent the second main port 309 thereof. Two pairs of stop members 385 extend from the instrument accommodating housing 305 of the adapter 301 adjacent the second main port 309 for abutting an outer surface 386 defined by the base plate 326 of the sleeve storing housing 315 to limit engagement movement of the sleeve storing housing 315 on the adapter 301. The pairs of the stop members 385 are arranged at 180° intervals around the adapter 301.

A pair of latching element 388 extend outwardly from the instrument accommodating housing 305, one latching element 388 extending spaced apart axially from the corresponding pair of stop member 385 between the corresponding pair of stop members 385 and the end 352 of the second main port 309. A pair of latch accommodating recesses 389 extend inwardly into the base plate 326 from the rim 343 defining the central access opening 327 at 180° intervals around the rim 343 for accommodating the latching elements 388 therethrough as the instrument accommodating housing 305 adjacent the second main port 309 is being engaged by the central access opening 327 of the first part 321 of the sleeve storing housing 315. A pair of receivers 390 located on an inner surface 391 adjacent the rim 343 defining the central access opening 327 in the base plate 326, are also spaced apart 180° around the rim 343 for engaging the latching elements 388. Once the latching elements 388 have passed through the latch accommodating recesses 389, the sleeve storing housing 315 is rotated through 90° relative to the second main port 309 of the adapter 301 for engaging the latching elements 388 with the receivers 390, for in turn tightly and securely but releasably engaging the sleeve storing housing 315 with the adapter 301.

A pair of limit members 392 extend from the outer surface 386 of the base plate 326 adjacent the rim 343 defining the central access opening 327 spaced apart at 180° around the rim 343 for engaging a corresponding ones of the pairs of stop members 389 of the pairs thereof in order to align the latching elements 388 respectively with the latch accommodating recesses 389 and the receivers 390.

In use, with the sleeve storing housing 315 releasably secured to the instrument accommodating housing 305 of the adapter 301 adjacent the second main port 309 by the latching elements 388 engaging the receivers 390, and with a filter (not shown) similar to the first filter 32 of the face mask described with reference to Figs. 1 to 6 sealably engaged in the air accommodating port 312 of the adapter 301, the instrument shielding apparatus 300 is ready for use. Initially, a distal end, for example, the distal end 83 of the endoscope 10 is entered into the instrument accommodating bore 335 of the sleeve storing housing 315 through the instrument receiving bore 330 of the end cap 329 and in turn through the cross-slit valve 382. The distal end 83 of the endoscope 10 is urged through the instrument accommodating bore 335 of the sleeve storing housing 315, and in turn into the second main port 309 of the adapter 301, and through the instrument accommodating bore 310 thereof, until the distal end 83 of the endoscope 10 extends through the first main port 307 of the adapter 301. As described with reference to the embodiments of the instrument shielding apparatus 91 the instrument shielding device 303 and the adapter 301 are urged proximally along the endoscope 10 until the diverging proximal end 58 of the endoscope 10 is tightly and sealably engaged by the cross-slit valve 382. The instrument shielding device 303 is then disengaged from the adapter 301 by rotating the adapter 301 through approximately 90° until the limit members 392 of the sleeve storing housing 315 are engaged by the corresponding stop members 385, at which stage the latching elements 388 are aligned with the respective latch accommodating recesses 389 in the base plate 326. The adapter 301 is then urged distally from the instrument shielding device 303 along the endoscope 10 towards the distal end 83 thereof. As the adapter 301 is being urged distally from the instrument shielding device 303, the shielding sleeve 317 is drawn from the annular storage chamber 340 through the annular outlet opening 345. Once the adapter 301 has been urged to the distal end 83 of the endoscope 10 with the distal end 83 thereof extending through the first main port 307, the first main port 307 is engaged in the instrument port of the mask. Thereafter, the mask is secured to the face of the subject and the endoscope 10 or other instrument is urged orally or nasally into the subject, as already described with reference to the instrument shielding apparatus 91.

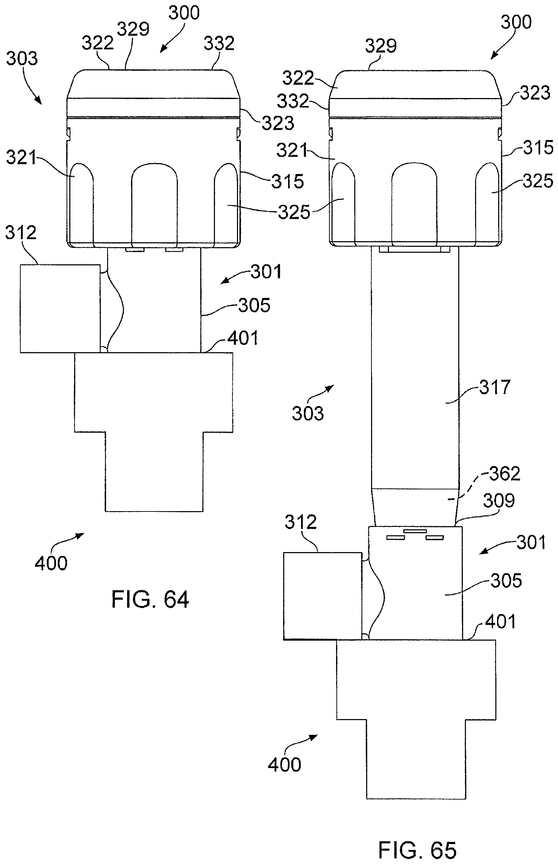

Referring now to Figs. 64 and 65 the instrument shielding apparatus 300 comprising the instrument shielding device 303 and the adapter 301 is illustrated secured to a trocar 400 adjacent an instrument port 401 of the trocar 400 for shielding an instrument, which is being entered through the trocar 400 into an insufflated cavity in a subject, for example, the peritoneal cavity, in which a minimal invasive procedure is being carried out laparoscopically or otherwise. In this embodiment of the invention the first main port 307 of the adapter 301 is releasably and sealably engaged in the instrument port 401 of the trocar 400. The air accommodating port 309 with the filter (not shown but similar to the filter 32 of the adapter described with reference to Figs. 1 to 6), may be used to accommodate insufflating gas from or to the cavity being insufflated, or if not required is closed-off by a suitable sealing plug sealably engaged in the air accommodating port 312.

Use of the instrument shielding apparatus 300 in conjunction with a trocar 400 is substantially similar to that described with reference to its use with a face mask. With the instrument shielding device 303 and the adapter 301 releasably secured together, the distal end of the instrument which is to be entered into the trocar 400 is entered initially into the instrument receiving bore 330, and in turn into the cross-slit valve 382, and in turn through the instrument accommodating bore 310 of the adapter 301. The instrument shielding device 303 and the adapter 301 still releasably secured together are urged proximally along the instrument, until the duckbill valve or other suitable valve or coupling element in the instrument accommodating bore 335 of the instrument shielding device 303 is sealably engaged on the instrument adjacent a proximal end thereof. The adapter 301 is then disengaged from the instrument shielding device 303 and is urged distally along the instrument until the distal end of the instrument is adjacent and extending through the first main port 307 of the adapter 301. The first main port 307 is then engaged in the instrument port 401 of the trocar 400. At this stage the portion of the instrument extending outwardly from the instrument port 401 of the trocar 400 to the proximal end of the instrument is shielded by the shielding sleeve 317.

Otherwise, use of the instrument shielding apparatus 300 in conjunction with a trocar 400 is similar to that described with reference to the use of the adapter 300 with a face mask.

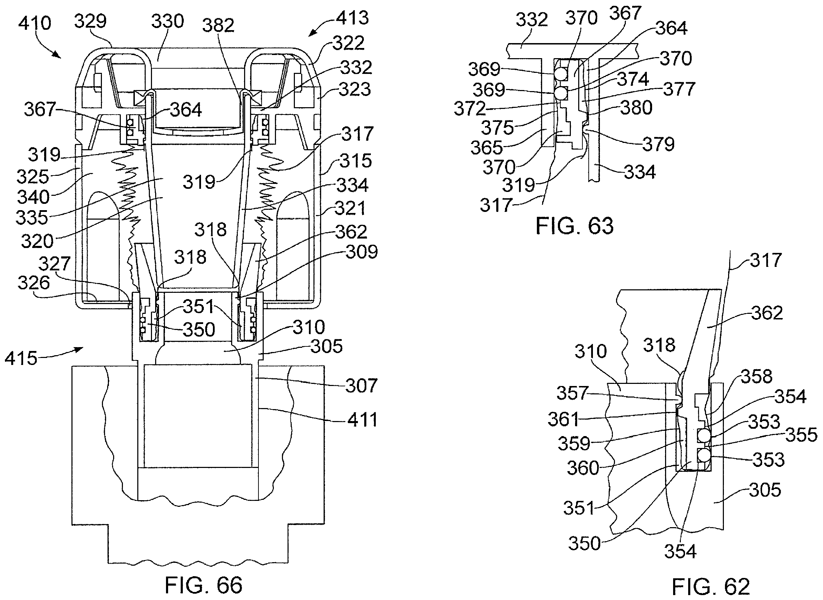

Referring now to Fig. 66 there is illustrated an instrument shielding apparatus according to another embodiment of the invention indicated generally by the reference numeral 410 for shielding a portion of an instrument (not shown), for example, a laparoscope extending from a proximal end of a trocar during a procedure being carried out, for example, laparoscopically in a cavity being insufflated in a human or animal subject. The instrument shielding apparatus 410 is illustrated in Fig. 66 sealably engaged in an instrument port 411 of a trocar 412, an upper portion only of which is illustrated. The trocar 412 would be used to access a cavity, for example, the peritoneal cavity in a subject, which is being insufflated, and in which an investigative or surgical procedure is being carried out. The instrument shielding apparatus 410 comprises an instrument shielding device 413 which is similar to the instrument shielding device 303 of the instrument shielding apparatus 300 described with reference to Figs. 51 to 65, and similar components are identified by the same reference numerals. In this embodiment of the invention the instrument shielding apparatus 410 comprises a port connector 415 for coupling the instrument shielding device 413 to the instrument port 411 of the trocar 412. In Fig. 66 the instrument shielding device 413 is illustrated with the shielding sleeve 317 in the stored state and with the sleeve storing housing 313 releasably engaged on the port connector 415.

Turning now in particular to the port connector 415, the port connector 415 is substantially similar to the adapter 301 of the instrument shielding apparatus 300, with the exception that the port connector 415 does not include an air accommodating port, nor does it include a first bore. Accordingly, in this embodiment of the invention the port connector 415 comprises a tubular instrument accommodating housing 305 having an instrument accommodating bore 310 extending therethrough from a first main port 307 to a second main port 309. The first main port 307 is adapted for engaging an instrument port in a trocar or a face mask, and in this case for engaging the instrument port 411 of the trocar 412. The second main port 309 is adapted for receiving an instrument from the instrument shielding device 413 into the instrument accommodating bore 310, and in turn into the trocar 412 through the instrument port 411 of the trocar 412. An annular receiving recess 351 extends into the instrument accommodating housing 305 around the second main port 309 and within which the first end 318 of the shielding sleeve 317 is secured to the second main port 309 by the coupling ring 350 in a similar manner as already described with reference to the instrument shielding device 303 of the instrument shielding apparatus 300. The sleeve storing housing 315 is releasably engageable with the second main port 309 of the port connector 415 in a similar manner as the sleeve storing housing 315 of the instrument shielding device 303 is releasably engageable with the adapter 301 of the instrument shielding apparatus 300.

Use of the instrument shielding apparatus 410 is similar to that of the instrument shielding apparatus 300. With the instrument shielding apparatus 410 disengaged from the trocar and with the instrument shielding device 413 releasably engaged with the second main port 309 of the port connector 415, the instrument, for example, a laparoscope, an endoscope or the like or other such instrument is entered into the instrument shielding device 413 through the instrument port 330 of the end cap 329, and in turn through the instrument accommodating bore 335 of the shield storing housing 315, and in turn through the instrument bore 310 of the port connector 415. The instrument shielding apparatus 410 is then urged proximally along the instrument to the proximal end of the instrument until the cross-slit valve 382 in the instrument shielding device 413 sealably engages the proximal end of the instrument. The port connector 415 is disengaged from the shield storing housing 315 of the instrument shielding device 412 and is urged distally along the instrument towards the distal end thereof. With the distal end of the instrument extending through the first main port 307 of the port connector 415, the first main port 307 is engaged in the instrument port 411 of the trocar 412. The instrument is then urged into and through the trocar 412 until the instrument reaches the cavity in which the procedure is being carried out. Thereafter use of the instrument shielding apparatus 410 is similar to that described with reference to the instrument shielding apparatus 300.

Otherwise, the instrument shielding apparatus 410 and its use is similar to that described with reference to the instrument shielding apparatus 300.

Since the portion of the instrument extending outwardly from the instrument port 411 of the trocar 412 to the proximal end of the instrument is now protected by the shielding sleeve 317, the surgeon grips the instrument through the shielding sleeve 317 in order to avoid contamination of the hands of the surgeon with pathogens as the instrument is manoeuvred inwardly and outwardly of the cavity, in which the procedure is being carried out in the subject. Accordingly, the hands of the surgeon carrying out the procedure are protected from any pathogens which may reside in the cavity of the subject, and which may be transferred onto the instrument.

Additionally, since the first main port 307 engages the instrument port 411 of the trocar 412 sealably, and since the first and second ends 318 and 319 of the shielding sleeve 317 are sealably engaged in the second main port 309 of the port connector 415 and in the intermediate plate member 332 of the sleeve storing housing 315, respectively, and since the proximal end of the instrument is sealably engaged by the cross-slit valve 382 of the instrument shielding device 413, any insufflating gases leaking from the cavity through the trocar 412 are contained within the shielding sleeve 317, and therefore, there is virtually little or no danger of any insufflating gases in which pathogens may be entrained which pass into the trocar leaking into the environment. Accordingly, the surgeon and any other personnel assisting in the procedure are protected from such pathogens.

It is envisaged that the instrument shielding apparatus 410 may be used in conjunction with a face mask. In which case, the first main port 307 of the port connector 415 would be engaged in the instrument port of the face mask. In order to allow the subject to breathe through the face mask, the breathing apparatus described with reference to Figs. 29 to 32 would be engaged in a secondary port of the face mask.

It is also envisaged that the port connector 415 may be adapted for use with the instrument shielding apparatus described with reference to Figs. 19 to 28 and 43 to 50, in which case, the second main port 309 of the port connector 415 would be adapted to receive the coupling ring 98 of the instrument shielding apparatus described with reference to Figs. 19 to 28 and 43 to 50. Accordingly, with the instrument shielding apparatus described with reference to Figs. 19 to 28 and 43 to 50 comprising the port connector 415 instead of the adapters, the instrument shielding apparatus described with reference to Figs. 19 to 28 and 43 to 50 could be coupled to an instrument port of a trocar or other access device.

While specific types of face masks have been described, it will be readily apparent to those skilled in the art that any suitable type of face mask may be provided, all that is required is that the face mask should have at least one single port suitable for accommodating an instrument, such as an endoscope.

It will also be appreciated that while a number of different embodiments of the adapter have been described, many other configurations of the adapter and instrument accommodating housings of the adapter are envisaged. For example, in the embodiments of the invention where the adapters are provided without the second bore, it is envisaged that the first non-return valve may be configured to permit fluid flow in the inward direction, whereby the first bore would be configured to accommodate air for inhaling by the subject, and in which case, it is envisaged that the first filter would be sealably coupled to a second port of the face mask through the second non-return valve, which would be configured to permit fluid flow in the outward direction through the second port of the face mask to accommodate air and other gases exhaled or otherwise expelled orally or nasally from the subject. Alternatively, the first non-return valve could be configured to permit fluid flow in the outward direction from the instrument bore to the first secondary port, and the first filter would be located communicating with the first bore, and the second non-return valve would be sealably located in a second port of the face mask, and configured for accommodating air in the inward direction for inhaling by the subject through the second port of the face mask.

While a number of adapters, breathing apparatus and instrument shielding apparatus and instrument shielding devices have been described in conjunction with different adapters, as well as different face masks and trocars, it will be readily apparent to those skilled in the art that any of the adapters may be used with any of the instrument shielding apparatus and any of the instrument shielding devices, and likewise, any of the face masks or trocars may be likewise used with any of the adapters, instrument shielding apparatus and instrument shielding devices.

It will also be appreciated that the port connector described with reference to Fig. 66 may be used with any of the instrument shielding apparatus and any of the instrument shielding devices for connecting any of the instrument shielding apparatus and any of the instrument shielding devices to any of the face masks or to any of the trocars.

Although not illustrated in all the embodiments of the invention which have been described, it is envisaged that a second filter for filtering air being inhaled by the subject may be provided, and the second filter would be located in the second bore or located for communicating with the second bore of the adapter. Typically, the second filter would be coupled to the second secondary port or the second non-return valve. Alternatively, in cases where air for inhaling by the subject is accommodated through a secondary port in the face mask, the second filter would be located to communicate with in the second secondary port of the face mask. In general, it is envisaged that the second filter would be configured to filter out pathogens such as infections and viruses from the air being inhaled by the subject, and typically, the second filter would be configured to filter out particles of size greater than 0.05 microns.

Additionally, while the housing of the adapter has been described as comprising a main tubular member and a first tubular member, and in some cases, a second tubular member, it is envisaged that the housing may be of any suitable construction and shape. It will also be appreciated that the non-return valves may be incorporated within the housing, and it is also envisaged that the first and second bores instead of communicating with the first main port through the instrument bore, the first and second bores may communicate directly with the first main port in the housing of the adapter.

It will also be appreciated that the instrument accommodating housing of the adapters may be of any other suitable shape and/or construction.

While a particular construction of sleeve storing housings for accommodating the shielding sleeve of the instrument shielding device have been described, any other suitable constructions of housing to form a storage chamber for the shielding sleeve may be provided. It is also envisaged that the sleeve storing housing for the instrument shielding device 54, instead of being engageable with the second main port of the housing of the adapter, the sleeve storing housing of the instrument shielding device 54 may be formed integrally with the housing of the adapter.

Needless to say, the shielding sleeve may be of any other suitable material besides that described, and while it is preferable that the material of the shielding sleeve be transparent it is not essential.

While the breathing apparatus has been described as comprising a second filter, it is envisaged that in some embodiments the second filter may be omitted.

Where an instrument is being entered through the face mask through a secondary port or other port other than the port into which the adapter, instrument shielding device or breathing apparatus is sealably engaged, in order to avoid or minimise leakage of air or other gases exhaled or otherwise expelled orally or nasally through the subject, it is envisaged that such a port in a face mask through which an instrument is being entered through the face mask will be provided with a suitable sealing means for sealably and slideably engaging the instrument as it is being urged through the port or ports of the face mask. In cases where a port or ports of the face mask are not required, in general, it is envisaged that they will be closed by a suitable sealable closure plug.

Needless to say, if in some of the embodiments according to the invention which have been described, the second main port or the instrument accommodating bore extending through the housing of the adapter have not been described as comprising a means for slideably and sealably engaging the instrument being urged through the instrument accommodating bore of the adapter, it will be readily understood that a suitable sealing means, in general, would be located in either the second main port, the instrument accommodating bore or indeed the first main port which would slideably and sealably engage the instrument therein.

While the instrument shielding apparatus, the adapters and the instrument shielding devices which have been described with reference to the drawings have been described coupled to a face mask, it is envisaged that the instrument shielding apparatus, the adapters and the instrument shielding devices, as the case may be, may be supplied separately without a face mask, and would be supplied to be suitable for engaging a port of a face mask, typically, an instrument port of the face mask. It is also envisaged that the instrument shielding device may be supplied separately of a face mask and separately of any of the adapters.

Additionally, it will be appreciated that while the instrument shielding device has been described for use with the adapter, it is envisaged that the instrument shielding device may be supplied separately of the adapter and the face mask, and in such case, the instrument shielding device may be provided for use with such an adapter or for use directly with a face mask. Where the instrument shielding device is provided for use directly with a face mask, it is envisaged that the first end of the shielding sleeve will terminate in a first coupling element suitable for releasable and sealable engagement with an appropriate port of the face mask. This first coupling element could either be configured to couple the sleeve storing housing of the instrument shielding device directly to the face mask, or could be provided as a separate port engaging element which would engage the relevant port of the face mask.

It is also envisaged that the instrument shielding devices may be supplied for use with other devices besides the adapters according to the invention and a face mask, and it is envisaged that the instrument shielding devices may be used, for example, in conjunction with a trocar for shielding an instrument being inserted through a trocar into a subject, in, for example, laparoscopic and other minimal invasive procedures.

While the adapter of the instrument shielding apparatus and the housing of the breathing apparatus have been described as being releasably engageable in the relevant port of the face masks, it is envisaged that in some embodiments of the invention the adapter of the instrument shielding apparatus and the housing of the breathing apparatus may be permanently secured in the face masks, and in which case, the face mask and the instrument shielding apparatus, or the face mask and the breathing apparatus, as the case may be, would be supplied as a single separate unit. It is also envisaged that instead of the instrument shielding device being releasably engageable with the adapter, the instrument shielding device may be permanently secured to the adapter or the instrument accommodating housing, and in which case, the adapter and the instrument shielding device would be provided as a single integral unit.

It is further envisaged that instead of the instrument shielding devices being releasably engageable with the adapters and the adapters or the instrument shielding devices being releasably engageable with the relevant port of the face mask, it is envisaged that the instrument shielding devices may be permanently secured to the adapters or the face masks, and that the adapter and/or the instrument shielding devices would be permanently secured to the face mask, and in which case, the face mask with the adapter and/or the instrument shielding devices would be provided as a single integral unit.

Additionally, in cases where the instrument shielding device is configured for direct engagement with the face mask, it is envisaged that instead of the instrument shielding device being releasably engageable with the relevant port of the face mask, the instrument shielding device may be permanently secured to the face mask, and in which case, the face mask and the instrument shielding device would be provided as a single integral unit.

While the face mask described with reference to Fig. 36 is the only one of the face masks which have been described as comprising a collector for collecting air and other gases escaping around the periphery of the face mask between the face mask and the face of the subject, it will be readily apparent to those skilled in the art that each of the face masks of each of the embodiments described may comprise such a collector.

While the embodiment of the face mask described with reference to Fig. 36 has been described with the collecting conduit being coupled to a filter and a vacuum pump, it is envisaged that in some embodiments of the invention the collecting conduit may be configured for coupling to an existing vacuum and filtration system in an operating theatre or other such theatre. Alternatively, if such an existing vacuum system did not include a filtration system, the collection conduit would be connected to the existing vacuum system through the third filter.

While first, second and third filters and the filters of specific types have been described, it will be readily apparent to those skilled in the art that any other suitable filters may be provided, and it will also be appreciated that the first, second and third filters may be the same or different. While it is preferable that all the filters be capable of filtering out particles of size greater than 0.05 microns, in some embodiments of the invention the particle size for which the filters may be configured to filter may be different. However, it is envisaged that the filters will be configured to filter out particles of size greater than 0.1 microns, and preferably, particles of size greater than 0.05 microns, and ideally, the filters would be configured to filter out particles of size greater than 0.04 microns.

While the sealing means for sealably engaging the instrument, for example, the endoscope, in the secondary instrument bore defined by the secondary tubular member of the sleeve storing housing of the instrument shielding device have been described as comprising a duckbill valve, any other suitable valve or sealing means may be used, for example, it is envisaged that instead of a duckbill valve, a multi-leaf valve or a cross-slit valve may be used, or indeed any other suitable sealing means or sealing valve.

While the first filters of the adapters have been described as being connected directly to the first non-return valve in both the adapters and the breathing apparatus, and the filter of the face masks have been described as being connected directly to the air accommodating port, it is envisaged that the first filters may be connected to the first non-return valve or to the first secondary port, or the filter of the face mask may be connected to the air accommodating port, as the case may be, through a flexible conduit. Similarly, the second filters may be connected to the second non-return valve or the second secondary port, as the case may be, through a flexible conduit.

It is also envisaged that the first and second non-return valves may be formed within the housing of the adapter in the first and second bores, respectively, and in which case the filters would be coupled to the first and second secondary ports, respectively, either directly or through a conduit, flexible or otherwise.

## Claims

1. An instrument shielding device for shielding an instrument, the instrument shielding device (54,92,245,303) comprising an elongated shielding sleeve (55,95,317) extending between a first end (56,96,318) and a second end (57,97,319) and defining an elongated instrument accommodating lumen (59,101,320) extending therethrough from the first end (56,96,318) to the second end (57,97,319) for accommodating the instrument therein, and a sleeve storing housing (60,93,315) defining a storage chamber (61,94,328) for storing the shielding sleeve in a stored state, one of the first and second ends of the shielding sleeve being sealably secured to the sleeve storing housing, a first coupling element (52,98) adapted to couple the first end (56,96,318) of the shielding sleeve to one of a face mask and a trocar, and a second coupling element (62,118) adapted to couple the second end of the shielding sleeve to the instrument, **characterised in that** the sleeve storing housing comprises a tubular member (75,109,334) extending through the storage chamber, and defining with the sleeve storing housing the storage chamber (61,94,328) of annular shape extending around the tubular member (75,109,334) for accommodating the shielding sleeve in the stored state with the tubular member extending into the instrument accommodating lumen (59,101,320) of the shielding sleeve, and the tubular member (75,109,334) defining an instrument accommodating bore (76,110,335) extending through the sleeve storing housing (60,93,315) for accommodating the instrument therethrough.

2. An instrument shielding device as claimed in Claim 1 **characterised in that** the one of the first and second ends of the shielding sleeve, which is sealably secured to the sleeve storing housing is sealably secured to the sleeve storing housing in the storage chamber thereof.

3. An instrument shielding device as claimed in Claim 1 or 2 **characterised in that** the first coupling element (52,98) is adapted to sealably couple the first end (56,96,318) of the shielding sleeve (55,95,317) to the one of the face mask and the trocar.

4. An instrument shielding device as claimed in any preceding claim **characterised in that** the first coupling element (52,98) is adapted to releasably couple the first end of the shielding sleeve to the one of the face mask and the trocar.

5. An instrument shielding device as claimed in any preceding claim **characterised in that** the second coupling element (62,118) is adapted to sealably couple the second end (57,97,319) of the shielding sleeve (55,95,317) to the instrument.

6. An instrument shielding device as claimed in any preceding claim **characterised in that** the second coupling element (62,118) is adapted to releasably couple the second end of the shielding sleeve to the instrument.

7. An instrument shielding device as claimed in any preceding claim **characterised in that** the second coupling element (62,118) is adapted to slideably engage the instrument.

8. An instrument shielding device as claimed in any preceding claim **characterised in that** the shielding sleeve is urgeable from a collapsed stored state, to an extended state for shielding the instrument.

9. An instrument shielding device as claimed in any preceding claim **characterised in that** the shielding sleeve comprises a flexible material.

10. An instrument shielding device as claimed in any preceding claim **characterised in that** the sleeve storing housing (60,93,315) comprises a closure member (78,114,248) for closing the storage chamber, the closure member having an access opening therethrough for accommodating the shielding sleeve therethrough from the storage chamber.

11. An instrument shielding device as claimed in Claim 10 **characterised in that** the access opening (79,115,254) in the closure member (78,114,248) is centrally aligned with the instrument accommodating bore (76,110,335) extending through the sleeve storing chamber, and the tubular member (75,109,334) extending through the storage chamber defines with a rim of the closure member defining the access opening (79,115,254) therein, an annular access opening (80,117,255) for accommodating the shielding sleeve from the storage chamber.

12. An instrument shielding device as claimed in any preceding claim **characterised in that** the second end of the shielding sleeve is sealably secured to the sleeve storing housing, and the second coupling element (62,118) is adapted for coupling the sleeve storing housing to the instrument.

13. An instrument shielding device as claimed in Claim 12 **characterised in that** the second coupling element (62,118) is located in the instrument accommodating bore (76,110,335) extending through the sleeve storing housing.

14. An instrument shielding device as claimed in any of Claims 1 to 11 **characterised in that** the first end of the shielding sleeve is secured to the sleeve storing housing, and the first coupling element (52,98) is adapted to couple the sleeve storing housing to the one of the face mask and the trocar.

15. An instrument shielding device as claimed in Claim 14 **characterised in that** the shielding sleeve is stored in the storage chamber with the second end thereof extending outwardly from the storage chamber through the access opening (79,115,254) to the second coupling element (62,118).

## Patentansprüche

1. Instrumentenabschirmvorrichtung zum Abschirmen eines Instruments, wobei die Instrumentenabschirmvorrichtung (54, 92, 245, 303) umfasst: eine längliche Abschirmhülse (55, 95, 317), die sich zwischen einem ersten Ende (56, 96, 318) und einem zweiten Ende (57, 97, 319) erstreckt und ein längliches Instrumentenaufnahmelumen (59, 101, 320) definiert, das sich durch sie hindurch von dem ersten Ende (56, 96, 318) zu dem zweiten Ende (57, 97, 319) erstreckt, um das Instrument darin unterzubringen, und ein Hülsenaufbewahrungsgehäuse (60, 93, 315), das eine Aufbewahrungskammer (61, 94, 328) zum Aufbewahren der Abschirmhülse in einem aufbewahrten Zustand definiert, wobei eines des ersten und des zweiten Endes der Abschirmhülse abdichtbar an dem Hülsenaufbewahrungsgehäuse befestigt ist, ein erstes Kopplungselement (52, 98), das eingerichtet ist, um das erste Ende (56, 96, 318) der Abschirmhülse mit einer Gesichtsmaske oder einem Trokar zu koppeln, und ein zweites Kopplungselement (62, 118), das eingerichtet ist, um das zweite Ende der Abschirmhülse mit dem Instrument zu koppeln, **dadurch gekennzeichnet, dass** das Hülsenaufbewahrungsgehäuse ein röhrenförmiges Element (75, 109, 334) umfasst, das sich durch die Aufbewahrungskammer hindurch erstreckt und mit dem Hülsenaufbewahrungsgehäuse die Aufbewahrungskammer (61, 94, 328) mit ringförmiger Gestalt definiert, die sich um das röhrenförmige Element (75, 109, 334) erstreckt, um die Abschirmhülse im aufbewahrten Zustand aufzunehmen, wobei sich das röhrenförmige Element in das Instrumentenaufnahmelumen (59, 101, 320) der Abschirmhülse erstreckt und das röhrenförmige Element (75, 109, 334) eine Instrumentenaufnahmebohrung (76, 110, 335) definiert, die sich durch das Hülsenaufbewahrungsgehäuse (60, 93, 315) erstreckt, um das Instrument durch sich hindurch aufzunehmen.

2. Instrumentenabschirmvorrichtung nach Anspruch 1, **dadurch gekennzeichnet, dass** das eine des ersten und des zweiten Endes der Abschirmhülse, das abdichtbar an dem Hülsenaufbewahrungsgehäuse befestigt ist, abdichtbar an dem Hülsenaufbewahrungsgehäuse in dessen Aufbewahrungskammer befestigt ist.

3. Instrumentenabschirmvorrichtung nach Anspruch 1 oder 2, **dadurch gekennzeichnet, dass** das erste Kopplungselement (52, 98) eingerichtet ist, um das erste Ende (56, 96, 318) der Abschirmhülse (55, 95, 317) mit der Gesichtsmaske oder dem Trokar abdichtend zu koppeln.

4. Instrumentenabschirmvorrichtung nach einem der vorhergehenden Ansprüche, **dadurch gekennzeichnet, dass** das erste Kopplungselement (52, 98) eingerichtet ist, um das das erste Ende der Abschirmhülse mit der Gesichtsmaske oder dem Trokar lösbar zu koppeln.

5. Instrumentenabschirmvorrichtung nach einem der vorhergehenden Ansprüche, **dadurch gekennzeichnet, dass** das zweite Kopplungselement (62, 118) eingerichtet ist, um das zweite Ende (57, 97, 319) der Abschirmhülse (55, 95, 317) abdichtbar mit dem Instrument zu verbinden.

6. Instrumentenabschirmvorrichtung nach einem der vorhergehenden Ansprüche, **dadurch gekennzeichnet, dass** das zweite Kopplungselement (62, 118) eingerichtet ist, um das zweite Ende der Abschirmhülse lösbar mit dem Instrument zu verbinden.

7. Instrumentenabschirmvorrichtung nach einem der vorhergehenden Ansprüche, **dadurch gekennzeichnet, dass** das zweite Kopplungselement (62, 118) eingerichtet ist, um gleitend mit dem Instrument in Eingriff gebracht zu werden.

8. Instrumentenabschirmvorrichtung nach einem der vorhergehenden Ansprüche, **dadurch gekennzeichnet, dass** die Abschirmhülse aus einem zusammengeklappten, aufbewahrten Zustand in einen ausgefahrenen Zustand zur Abschirmung des Instruments drängbar ist.

9. Instrumentenabschirmvorrichtung nach einem der vorhergehenden Ansprüche, **dadurch gekennzeichnet, dass** die Abschirmhülse ein flexibles Material umfasst.

10. Instrumentenabschirmvorrichtung nach einem der vorhergehenden Ansprüche, **dadurch gekennzeichnet, dass** das Hülsenaufbewahrungsgehäuse (60, 93, 315) ein Verschlusselement (78, 114, 248) zum Verschließen der Aufbewahrungskammer umfasst, wobei das Verschlusselement eine Zugangsöffnung zum Aufnehmen der Abschirmhülse aus der Aufbewahrungskammer durch sich hindurch aufweist.

11. Instrumentenabschirmvorrichtung nach Anspruch 10, **dadurch gekennzeichnet, dass** die Zugangsöffnung (79, 115, 254) in dem Verschlusselement (78, 114, 248) mittig mit der sich durch die Hülsenaufbewahrungskammer erstreckenden Instrumentenaufnahmebohrung (76, 110, 335) ausgerichtet ist und das sich durch die Aufbewahrungskammer erstreckende röhrenförmige Element (75, 109, 334) mit einem Rand des Verschlusselements, der die Zugangsöffnung (79, 115, 254) darin definiert, eine ringförmige Zugangsöffnung (80, 117, 255) zur Aufnahme der Abschirmhülse aus der Aufbewahrungskammer definiert.

12. Instrumentenabschirmvorrichtung nach einem der vorhergehenden Ansprüche, **dadurch gekennzeichnet, dass** das zweite Ende der Abschirmhülse abdichtbar an dem Hülsenaufbewahrungsgehäuse befestigt ist und das zweite Kopplungselement (62, 118) eingerichtet ist, um das Hülsenaufbewahrungsgehäuse mit dem Instrument zu koppeln.

13. Vorrichtung zur Abschirmung eines Instruments nach Anspruch 12, **dadurch gekennzeichnet, dass** das zweite Kopplungselement (62, 118) in der sich durch das Hülsenaufbewahrungsgehäuse erstreckenden Instrumentenaufnahmebohrung (76, 110, 335) angeordnet ist.

14. Instrumentenabschirmvorrichtung nach einem der Ansprüche 1 bis 11, **dadurch gekennzeichnet, dass** das erste Ende der Abschirmhülse an dem Hülsenaufbewahrungsgehäuse befestigt ist und das erste Kopplungselement (52, 98) eingerichtet ist, um das Hülsenaufbewahrungsgehäuse entweder mit der Gesichtsmaske oder mit dem Trokar zu koppeln.

15. Instrumentenabschirmvorrichtung nach Anspruch 14, **dadurch gekennzeichnet, dass** die Abschirmhülse in der Aufbewahrungskammer aufbewahrt wird, wobei sich ihr zweites Ende aus der Aufbewahrungskammer nach außen durch die Zugangsöffnung (79, 115, 254) zu dem zweiten Kopplungselement (62, 118) erstreckt.

## Revendications

1. Dispositif de protection d'instrument pour protéger un instrument, le dispositif de protection d'instrument (54,92,245,303) comprenant un manchon de protection allongé (55,95,317) s'étendant entre une première extrémité (56,96,318) et une seconde extrémité (57,97,319) et définissant une lumière allongée (59,101,320) s'étendant à travers lui de la première extrémité (56,96,318) à la seconde extrémité (57,97,319) pour y loger l'instrument, et un boîtier de stockage du manchon (60,93,315) définissant une chambre de stockage (61,94,328) pour stocker le manchon de protection à l'état stocké, l'une des première et deuxième extrémités du manchon de protection étant fixée de manière étanche au boîtier de stockage du manchon, un premier élément de couplage (52,98) adapté pour coupler la première extrémité (56,96,318) du manchon de protection à un masque facial ou à un trocart, et un second élément de couplage (62,118) adapté pour coupler la seconde extrémité du manchon de protection à l'instrument, **caractérisé en ce que** le boîtier de stockage du manchon comprend un élément tubulaire (75,109,334) s'étendant à travers la chambre de stockage, et définissant avec le boîtier de stockage du manchon la chambre de stockage (61,94,328) de forme annulaire s'étendant autour de l'élément tubulaire (75,109,334) pour loger le manchon de protection à l'état stocké, l'élément tubulaire s'étendant dans la lumière (59,101,320) du manchon de protection, et l'élément tubulaire (75,109,334) définissant un alésage (76,110,335) s'étendant à travers le logement de stockage du manchon (60,93,315) pour loger l'instrument à travers ce dernier.

2. Dispositif de protection d'un instrument selon la revendication 1, **caractérisé en ce que** la première et la deuxième extrémité du manchon de protection, qui est fixée de manière étanche au boîtier de stockage du manchon, est fixée de manière étanche au boîtier de stockage du manchon dans la chambre de stockage de ce dernier.

3. Dispositif de protection d'un instrument selon la revendication 1 ou 2, **caractérisé en ce que** le premier élément de couplage (52,98) est adapté pour coupler de manière étanche la première extrémité (56,96,318) du manchon de protection (55,95,317) au masque facial ou au trocart.

4. Dispositif de protection d'un instrument selon l'une des revendications précédentes, **caractérisé en ce que** le premier élément de couplage (52,98) est adapté pour relier de manière amovible la première extrémité du manchon de protection au masque facial ou au trocart.

5. Dispositif de protection d'un instrument selon l'une quelconque des revendications précédentes, **caractérisé en ce que** le second élément de couplage (62,118) est adapté pour coupler de manière étanche la seconde extrémité (57,97,319) du manchon de protection (55,95,317) à l'instrument.

6. Dispositif de protection d'un instrument selon l'une quelconque des revendications précédentes, **caractérisé en ce que** le second élément de couplage (62,118) est adapté pour coupler de manière amovible la seconde extrémité du manchon de protection à l'instrument.

7. Dispositif de protection d'un instrument selon l'une quelconque des revendications précédentes, **caractérisé en ce que** le second élément de couplage (62,118) est conçu pour s'engager de manière coulissante avec l'instrument.

8. Dispositif de protection d'un instrument selon l'une des revendications précédentes, **caractérisé en ce que** le manchon de protection peut être poussé d'un état de stockage replié à un état étendu pour protéger l'instrument.

9. Dispositif de protection d'un instrument selon l'une des revendications précédentes, **caractérisé en ce que** le manchon de protection comprend un matériau souple.

10. Dispositif de protection d'un instrument selon l'une quelconque des revendications précédentes, **caractérisé en ce que** le boîtier de stockage du manchon (60,93,315) comprend un élément de fermeture (78,114,248) pour fermer la chambre de stockage, l'élément de fermeture ayant une ouverture d'accès traversante pour recevoir le manchon de protection à travers celle-ci à partir de la chambre de stockage.

11. Dispositif de protection d'un instrument selon la revendication 10, **caractérisé en ce que** l'ouverture d'accès (79,115,254) dans l'élément de fermeture (78,114,248) est alignée au centre avec l'ouverture de réception de l'instrument (76,110,335) s'étendant à travers la chambre de stockage du manchon, et l'élément tubulaire (75,109,334) s'étendant à travers la chambre de stockage définit avec un bord de l'élément de fermeture définissant l'ouverture d'accès (79,115,254) dans celui-ci, une ouverture d'accès annulaire (80,117,255) pour loger le manchon de protection à partir de la chambre de stockage.

12. Dispositif de protection d'un instrument selon l'une quelconque des revendications précédentes, **caractérisé en ce que** la seconde extrémité du manchon de protection est fixée de manière étanche au boîtier de stockage du manchon, et que le second élément de couplage (62,118) est adapté pour coupler le boîtier de stockage du manchon à l'instrument.

13. Dispositif de protection d'un instrument selon la revendication 12, **caractérisé en ce que** le second élément de couplage (62,118) est situé dans l'ouverture de réception de l'instrument (76,110,335) qui s'étend à travers le boîtier de stockage du manchon.

14. Dispositif de protection d'un instrument selon l'une des revendications 1 à 11, **caractérisé en ce que** la première extrémité du manchon de protection est fixée au boîtier de stockage du manchon, et que le premier élément de couplage (52,98) est adapté pour coupler le boîtier de stockage du manchon au masque facial ou au trocart.

15. Dispositif de protection d'un instrument selon la revendication 14, **caractérisé en ce que** le manchon de protection est stocké dans la chambre de stockage, sa deuxième extrémité s'étendant vers l'extérieur de la chambre de stockage à travers l'ouverture d'accès (79,115,254) jusqu'au deuxième élément de couplage (62,118).
